# EUROPEAN PATENT SPECIFICATION

(11) **EP 1 960 076 B1**
(45) Date of publication and mention of the grant of the patent: **16.04.2014**
(21) Application number: 06800435.7
(22) Date of filing: 26.07.2006
(51) Int. Cl.: A61L 11/00, B01D 11/02

(54) **APPARATUS FOR HOSPITAL WASTE TREATMENT**
VORRICHTUNG ZUR BEHANDLUNG VON KRANKENHAUSABFALL
APPARRAIL POR LE TRAITEMENT DES DECHETS EN MILIEU HOSPITALIER

(30) Priority: 26.07.2005 US 190343; 25.08.2005 US 212009
(43) Date of publication of application: 27.08.2008
(73) Proprietor: Trinova Medical Waste Solutions, Long Beach CA 90813 (US)
(72) Inventor: Jakobi, Felix F., Long Beach, CA 90803 (US); Bennett, James F., La Mirada, CA 90638 (US)
(74) Representative: Purdy, Hugh Barry
(86) International application number: PCT/US2006/029346
(87) International publication number: WO 2007/014312

(56) References cited:
- US-A- 5 116 574
- US-A- 5 207 994
- US-A- 5 364 589
- US-A- 5 614 157
- US-A- 5 656 248
- US-A- 5 820 541

## Description

### TECHNICAL FIELD

The present invention relates to a device and method for treatment of waste and in particular to the treatment of infectious waste from a hospital.

### BACKGROUND ART

In the normal course of operation, hospitals generate a variety of waste which is not suitable for normal disposal. While some or most hospital waste may be harmless, it is difficult to distinguish such harmless waste from infectious waste. As a result, all of the waste from a hospital must be treated as if it may be harmful. Also, sensitivity to the handling of hospital waste has been raised as a result of AIDS and other health issues. Recently, the bird flu spread rapidly and initially was not well understood. As world travel has increased, so has the ability of infections, like the bird flu, to spread rapidly, and the need to contain outbreaks is greater than ever before. For all of these reasons, there is a need to deal properly with hospital waste.

Common methods of treating hospital waste include systems having a steam autoclave or an ethylene oxide autoclave. U.S. Patent No. 6,726,136 for "Waste treatment plant," describes a system including an autoclave. Other systems include incinerators. Unfortunately, incinerators may be difficult to construct and operate, and may create environmental issues. Autoclaves may also be expensive and difficult to operate. Systems including autoclaves may also require additional steps to complete disinfecting waste.

U.S. Patent Nos. 5,425,925 and 5,656,248 for "Multi-stage infectious waste treatment system," both assigned to the assignee of the present application, describe waste treatment systems which use grinders to grind waste into small particle size, and then soak the waste in a volatile liquid disinfectant. Unfortunately, while the systems described in the '925 and the '248 patents successfully treat most hospital waste, some hospital waste has been found to contain material, such as titanium prosthetic joints, which may jam known waste grinders. The '925 and the '248 patents are herein incorporated by reference.

US Patent Application No. 11/190,343 filed July 26, 2005 for "INFECTIOUS WASTE TREATMENT" filed by the inventor of the present patent application describes a hospital waste disposal system including a shredder which addresses many of the issues of the '925 and the '248 patents, but unfortunately, a remaining problem is a failure to shred all of the hospital waste components into small enough elements to pass through pumps and other system components. Some hospital waste, such as titanium pins, large needles, medical drills, and the like, may be bent and twisted by the shredder, but not cut or shredded into small enough pieces. In particular, these bent and twisted pins may foul or jam a pump used to advance the shredded and wetted waste through the hospital waste treatment system and may foul or jam other pumps used to circulate the liquid disinfectant. The '343 application is herein incorporated by reference.

US Patent Application No. 11/212,009, filed August 25, 2005, for "HOSPITAL WASTE TREATMENT WITH IMPROVED DISINFECTANT LIQUID PRODUCTION" filed by the inventor of the present invention, describes a hospital waste disposal system including an improved liquid disinfectant generation system, but did not address the fouling or jamming of pumps used to circulate the liquid disinfectant. The '343 application is herein incorporated by reference.

### DISCLOSURE OF THE INVENTION

The present invention addresses the above and other needs by providing a waste treatment system as defined according to claim 1.

In accordance with a first embodiment of the invention, there is provided a first apparatus for infectious waste treatment. The apparatus comprises a lift for lifting a waste container to a feed hopper which receives waste material from the waste container, a shredder for receiving the waste material from the feed hopper and shredding the waste material, a wetting area comprising a main solution tank for receiving and wetting the shredded waste material, and a dwell area comprising an auger for carrying the wetted material from the main solution tank. The shredder comprises a rotor positioned below the feed hopper, an anvil in shredding cooperation with the rotor, and a sizing screen for controlling the size of the shredded waste material. A liquid disinfectant is sprayed onto the shredded waste material in the main solution tank, and a chopper pump circulates the liquid disinfectant. The auger carries the wetted slurry from the main solution tank and provides a dwell time. A de-watering section resides at the end of the auger.

In accordance with a second embodiment of the invention, there is provided a second apparatus for infectious waste treatment. Unprocessed waste material is dumped into a feed hopper. The feed hopper feeds the unprocessed waste material into a shredder. The feed hopper includes a hopper ram for pushing the waste material into a rotor and anvil for shredding the unprocessed waste material. The shredded material falls into a wetting area comprising a treatment hopper where the shredded material is mixed with liquid disinfectant to form a slurry. The slurry material is drawn from the treatment hopper and advanced into a dwell area comprising a coil by an air operated pump, and spends three to four minutes of dwell time in the coil before entering a de-watering system. A preferred de-watering system includes a piston which compresses the waste in the de-watering system and the liquid disinfectant escapes through straining holes which filter the liquid disinfectant for re-use. The de-watered material is then dumped from the de-watering system and carried away for disposal.

### BRIEF DESCRIPTION OF THE DRAWING

The above and other aspects, features and advantages of the present invention will be more apparent from the following more particular description thereof, presented in conjunction with the following drawings wherein:
FIG. 1 is a first embodiment of a waste treatment system according to the present invention.
FIG. 2A shows a lift according to the present invention for lifting a waste container to dump waste material carried by the waste container into a feed hopper of the waste treatment system.
FIG. 2B shows the waste being dumped into the feed hopper.
FIG. 3A shows a side view of a shredder suitable for use with the waste treatment system according to the present invention.
FIG. 3B shows a top view of the shredder.
FIG. 3C shows an end view of the shredder (note the shredder resides sideways in the waste treatment system.)
FIG. 4 is a cross-sectional view of the shredder taken along line 4-4 of FIG. 313.
FIG. 5 is a cross-sectional view of the shredder taken along line 5-5 of FIG. 313.
FIG. 6A is a side view of a main solution tank according to the present invention suitable for use with the waste treatment system.
FIG. 6B is a top view of the main solution tank.
FIG. 7 is a cross-sectional view of the main solution tank taken along line 7-7 of FIG. 6B.
FIG. 8 is a second side view of the main solution tank (opposite side) showing a gas monitoring system according to the present invention, a chopper and recirculation pump, and a liquid disinfectant generator according to the present invention.
FIG. 9 is a detailed view of a chemical manifold according to the present invention.
FIG. 10 is a second embodiment of a waste treatment system according to the present invention.
FIG. 11A shows a lift according to the present invention for lifting a waste container to dump waste material carried by the waste container into a feed hopper according to the present invention.
FIG. 11B shows the waste container with waste being poured into the second waste treatment system.
FIG. 11C is a side view of a feed hopper lid according to the present invention.
FIG. 11D is a cross-sectional view of the lid taken along line 11D-11D of FIG. 11C showing hopper ram according to the present invention, for pushing waste into the shredder.
FIG. 12 is a top view of a treatment hopper according to the present invention, for mixing liquid disinfectant with shredded waste material.
FIG. 13 is a detailed view of a main pump according to the present invention used to advance wetted slurry through the waste treatment system.
FIG. 13A is a side view of a gate valve according to the present invention.
FIG. 13B is a front view of the gate valve.
FIG. 14A is a cross-sectional view of gate valve taken along line 14-14 of FIG. 13A with the gate valve closed.
FIG. 14B is a cross-sectional view of gate valve taken along line 14-14 of FIG. 13A with the gate valve open.
FIG. 15 is a cross-sectional view of gate valve taken along line 15-15 of FIG. 13B with the gate valve closed.
FIG. 16 is a de-watering system according to the present invention.
FIG. 16A is a cross-sectional view of the de-watering system taken along line 16A-16A of FIG. 16.
FIG. 17 is a main tank and waste water tank according to the present invention.
FIG. 18 is a chlorine generation system according to the present invention.
FIG. 19 is a chlorine dioxide monitoring system according to the present invention.
FIG. 20 is an overview of liquid disinfectant flow according to the present invention within the waste treatment system.
FIG. 21 is an add-on paper shredder system according to the present invention.
FIG. 22 is a method of waste treatment according to the present invention.

Corresponding reference characters indicate corresponding components throughout the several views of the drawings.

### MODES FOR CARRYING OUT THE INVENTION

The following description is of the best mode presently contemplated for carrying out the invention. This description is not to be taken in a limiting sense, but is made merely for the purpose of describing one or more preferred embodiments of the invention. The scope of the invention should be determined with reference to the claims.

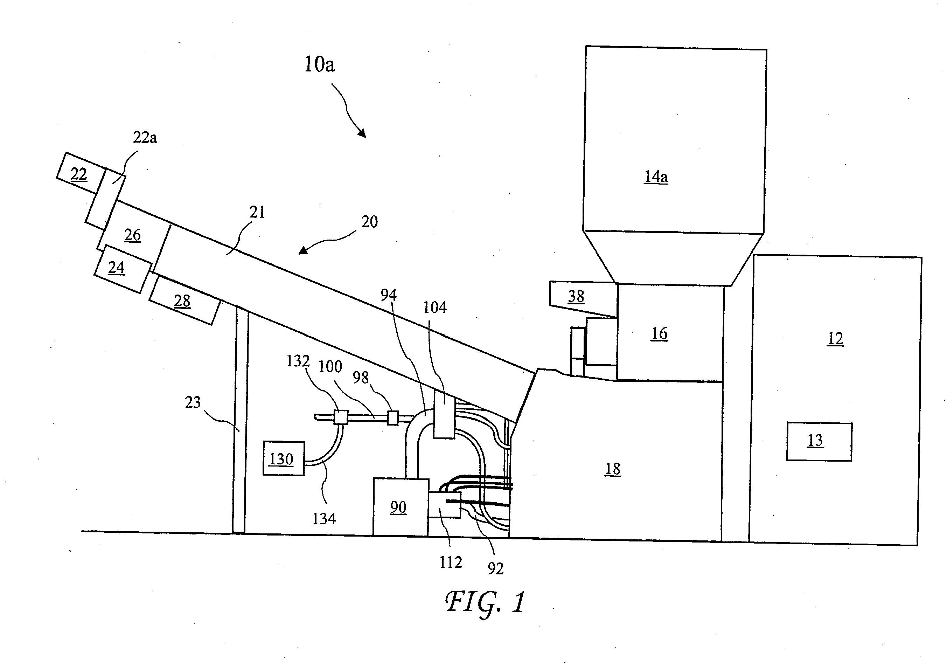

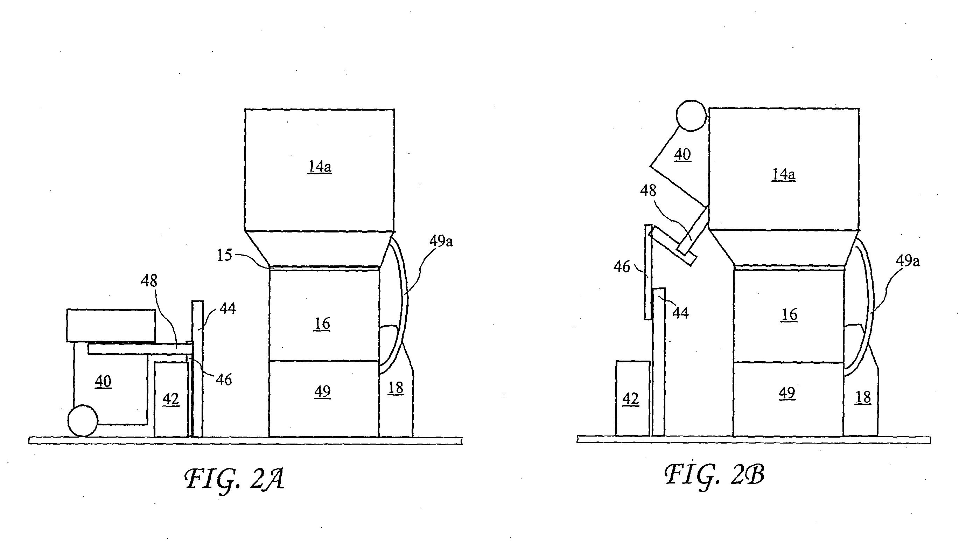

A first embodiment of a waste treatment system 10a according to the present invention is shown in FIG. 1. The waste treatment system 10a includes a cage 12, first feed hopper 14a, a shredder 16, a wetting area comprising a main solution tank 18, and a dwell area comprising an auger 20. A hospital waste container 40 (see FIGS. 2A and 2B) is placed into the cage 12 where a lift unit 42 lifts the container 40 and dumps hospital waste carried in the container 40 into the feed hopper 14a (see FIGS. 2A and 2B). The feed hopper 14a resides above the shredder 16 and feeds the waste into the shredder 16. The shredder 16 shreds the waste, and the shredded waste drops into the main solution tank 18 where the shredded waste is wetted in a disinfectant liquid to create a wetted slurry. The auger 20 is in fluid cooperation with the main solution tank 18 and lifts the wetted slurry from the main solution tank 18 and completes the waste treatment.

A radioactive material detector 13 resides in the cage 12. When the radioactive material detector 13 detects radiation in the hospital waste, the waste treatment system 10a is turned off and an alarm is sounded. An example of a suitable radioactive material detector is Micro Bomb Detector made by AI NOTL Systems Inc. in Ontario, Canada.

Continuing with FIG. 1, a pump 90 receives disinfectant liquid from the main solution tank 18 through a pump inlet line 92, and returns the disinfectant liquid through a pump outlet line 94 through a manifold 104. A drain line 100 is connected to the pump outlet line 94 through a drain valve 98. A neutralizer tank 130 is connected to the drain line 100 at a neutralizer injector 130 for neutralizing the drained disinfectant liquid. The pump 90 is preferably a chopper pump, and is more preferably a high flow rate pump, and most preferably an approximately 200 Gallon Per Minute (GPM) pump. An example of a suitable 200 GPM pump is a model number HE3G6SEC-055 chopper pump manufactured by Vaughn Company in Montesano, Washington. In some cases, two separate pumps may be used to recycle the disinfectant liquid and to spray the disinfectant liquid onto the waste material. When two pumps are used, the pumps are preferably approximately 90 Gallon Per Minute (GPM) pumps.

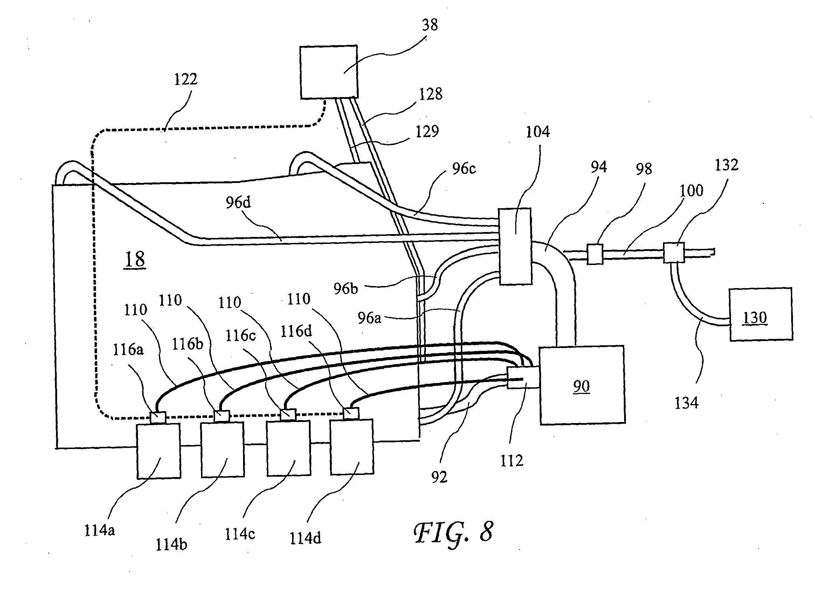

A continuous gas monitoring system 38 monitors the liquid disinfectant level in the main solution tank 18 and composition (i.e., strength) of the liquid disinfectant, and controls the generation of liquid disinfectant (see FIG. 8). For example, chemicals may be introduced into a flow into the pump 90 at a chemical manifold 112 to generate liquid disinfectant. An example of a continuous gas monitoring system 38 is the system described in US Patent No. 5,269,832 for "Method and Apparatus for Continuously Measuring the Concentration of Chemicals in Solution." The '832 patent is herein incorporated by reference.

The auger 20 is preferably a shaftless auger residing in an auger housing 21 supported by an auger strut 23 and is powered by an auger motor 22 which is preferably connected to the auger 20 through a gearbox 22a. A de-wetting area is fitted to the auger 20, wherein the liquid disinfectant used to wet the shredded waste is trapped and recirculated back into the main tank. The de-wetting area is in fluid cooperation with the auger 20 and comprises a rotatable section 26 of the auger housing 21 which may be rotationally positioned relative to the auger housing 21 at various rotations to adjust the position of a chute 24 and a fluid trap 28. If the chute 24 is pointed down, the back pressure on the flow of the wetted slurry is minimized, and the amount of liquid disinfectant removed by the fluid trap 28 is minimized. As the chute 24 is rotated away from a pointed down position, the back pressure on the flow of the wetted slurry is increased, and the amount of liquid disinfectant removed by the fluid trap 28 is increased. If the chute 24 is rotated to an upward position, the back pressure on the flow of the wetted slurry is maximized, and the amount of liquid disinfectant removed by the fluid trap 28 is maximized.

A lift 42 for lifting a waste container, for example a wheeled bin 40, to dump waste into the feed hopper 14a of the waste treatment system 10a is shown in FIG. 2A. Grasping arms 48 are attached to a lift trolley 46 which travels on tracks 44. FIG. 2B shows the wheeled bin 40 grasped and lifted by the grasping arms 48, and the waste being dumped into the feed hopper 14a. A filter system 49 is connected to the feed hopper 14a by a filter hose 49a. The filter system 49 includes a fan to draw air from the feed hopper 14a, and preferably includes a High Efficiency Particle Arresting (HEPA) filter.

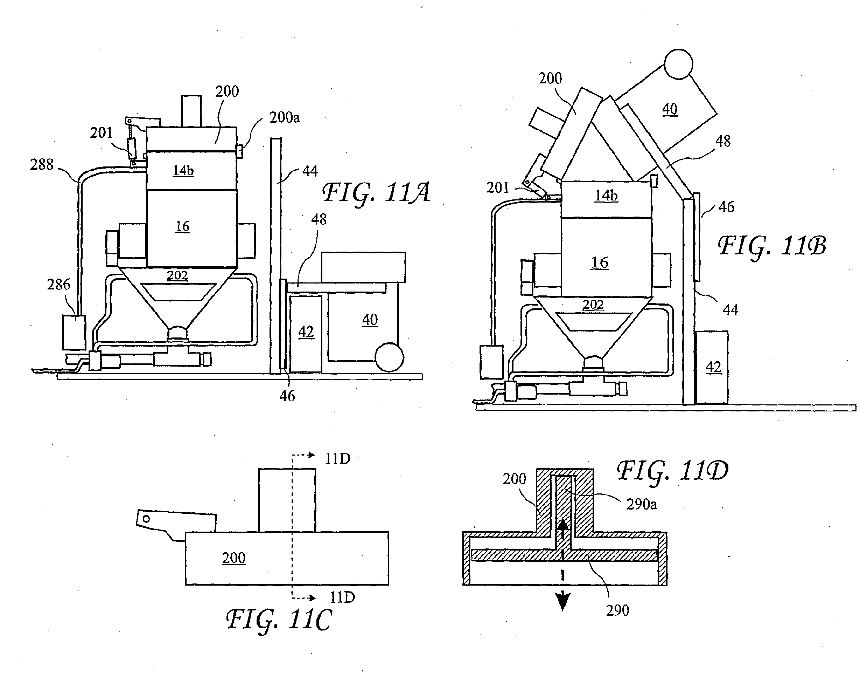

The lift 42 may be electrically, hydraulically, or pneumatically operated. The lift 42 preferably can lift a 95 US gallon, (360 Liter) and 65 US gallon (240 Liter) wheeled bin 40 vertically and empty its contents into the feed hopper 14a. Any containers too large to fit into a 65 US gallon wheeled bin 40 (i.e. greater than 20 inches by 20 inches) are preferably capable of being lifted by the lift mechanism. Plastic bags containing medical waste may be presented to the machine in either a 65 or 95 US gallon wheeled bin 40. The lift 42 is preferably capable of lifting up to 225 Lbs. with a cycle time of no more than 10 seconds and have its movement controlled by a Programable Logic Controller (PLC) to allow the wheeled bin 40 to be rocked and held stationery during any part of its lift cycle. The lift 42 preferably includes weight measuring in both pounds and kilograms and to an accuracy of approximately 0.5 %. The lift 42 preferably includes a Geiger counter for measuring any radiation from the waste load being lifted into the machine and is preferably capable of detecting alpha beta and gamma radiation and have an operating range of mR/hr: .001-50.00 mR/hr (Cs-137); CPM: 0-50,000; Total: 0-60,000 counts and to a Sensitivity: 1000 cpm/mR/hr referenced to Cs-137 and Accuracy: ±10% typical; ± 15 % max. The lift 42 is preferably also capable of supporting European SI units (mSv/hr). Both the weighing scales and Geiger counter are preferably supplied with standard parts for calibration. A Human Machine Interface (HMI) preferably resides beside the lift 42 and is preferably capable of supporting in-put from a bar code reader, or equivalent electronic reader. The HMI is further preferably capable of supporting direct data in-put via touch screen or keypad. A mechanical barrier is preferably provided to protect the lift 42 mechanism per OSHA or OSHPD standards, to prevent anyone from accessing the lift during any part of the lift process. During lift 42 operation, the shredder is automatically operated for a predetermined period which will be software configurable. A combined bleach and fresh water (ratio 1:1) spray point, for wash-down, is be fitted to the area beside the lift mechanism. A nozzle sprays a water bleach mixture into the feed hopper 14a approximately six inches from the top of the feed hopper and is active when the lid is closed. See FIG 11A. The system is designed to prevent material or splashes of liquid coming either out of the hopper or bin into the surrounding area using splash proof mechanical seals.

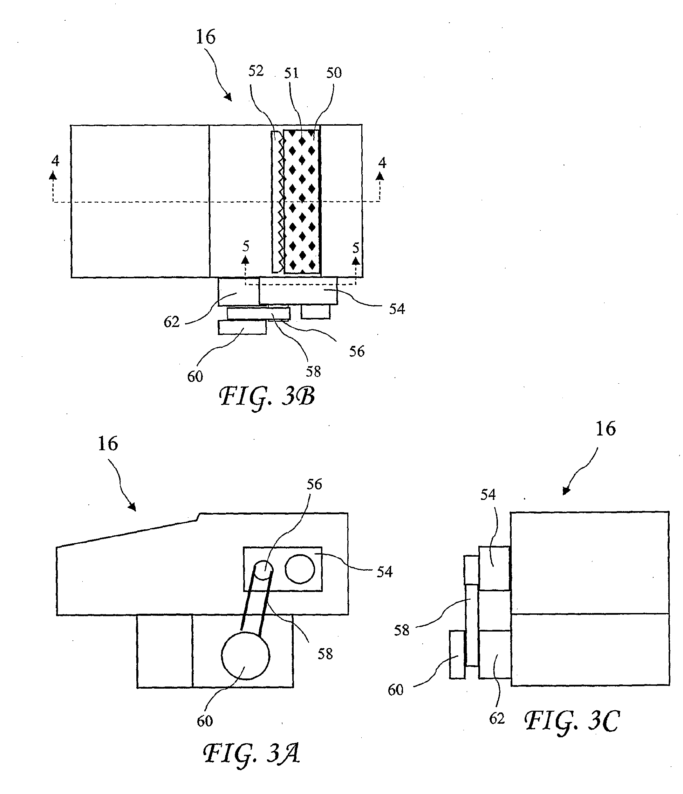

A side view of a shredder 16 suitable for use with the waste treatment system 10a is shown in FIG. 3A, a top view of the shredder 16 is shown in FIG. 3B, and an end view of the shredder (note the shredder resides sideways in the waste treatment system) is shown in FIG. 3C. The shredder 16 includes a rotor 50 having teeth 51 and is preferably capable of shredding approximately 1,500 Lbs. of waste per hour. The teeth 51 are preferably hardened D2 steel teeth. An anvil 52 cooperates with the rotor 50 to shred the waste material. Power is provided to the rotor by a shredder motor 62. A fluid coupling 60 connects the motor 62 to a belt 58. The belt 58 connects the fluid coupling 60 to a hub 56 on a shredder gear box 54, and the gear box 54 contains gears connecting the hub 56 to the rotor 50.

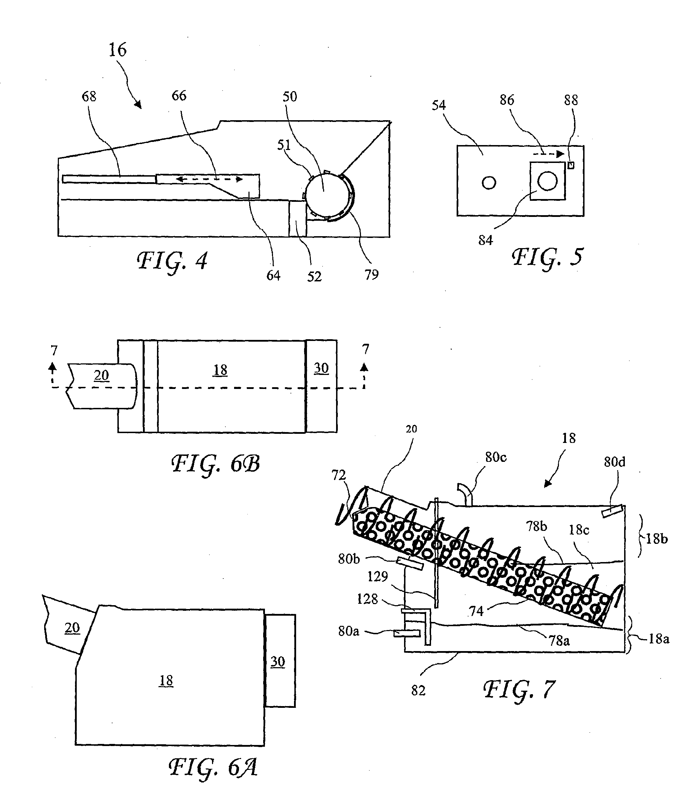

A cross-sectional view of the shredder 16 taken along line 4-4 of FIG. 3B is shown in FIG. 4. The rotor 50, teeth 51, and anvil 52 are fed by a shredder ram 64, which is preferably a hydraulic ram connected to hydraulics 68. The ram 64 moves toward and away from the rotor 50 as shown by arrow 66. The ram 64 is controlled to provide efficient operation of the rotor 50 and anvil 52, for example, if the ram 66 senses high resistance to motion toward the rotor 50, the speed of the ram 66 is reduced, and if the ram 66 senses low resistance to motion toward the rotor 50, the speed of the ram 66 is increased. A sizing screen 79 resides under the rotor 50, thereby limiting the maximum size of shredded waste material which may fall into the main solution tank 18. The sizing screen 79 may be selected with a hole size to control the size of the shredded material, the holes are preferably between approximately 1/2 inch diameter and approximately three inch diameter, and more preferably approximately 0.75 inches in diameter. The shredder 16 preferably shreds any items passed through it to the extent that the items may not be re-used. The shredder 16 further preferably shreds any items passed through it to have a maximum length of approximately six inches.

A cross-sectional view of the gearbox 54 taken along line 5-5 of FIG. 313 is shown in FIG. 5. The rotor 50 and gearbox 54 are mounted to the shredder 16 to allow motion 86 of the rotor 50 and gearbox 54 if sufficient force is exerted on the rotor 50 by waste material caught between the rotor 50 and the anvil 52, thus moving the rotor 50 away from the anvil 52. If the motion 86 is sufficiently large, a switch 88 turns the motor 62 off to avoid damage to the shredder 16.

A side view of the main solution tank 18 suitable for use with the waste treatment system 10a is shown in FIG. 6A, a top view of the main solution tank 18 is shown in FIG. 6B. A cross-sectional view of the main solution tank 18 taken along line 7-7 of FIG. 6B is shown in FIG. 7. An auger screw 72 extends though the main solution tank 18 and is cupped by an auger floor 74 which is preferably an auger screen extending under approximately half of the circumference of the auger screw 72. The liquid disinfectant resides in a lower portion 18a of the main solution tank 18 with a static fluid level 78a. Additionally, while the waste treatment system 10a is in operation, the liquid disinfectant resides at a dynamic level 78b above the auger floor 74 in a wetting portion 18c of the main solution tank 18. The dynamic liquid level 78b is maintained in equilibrium by the cooperation of pumping the liquid disinfectant into an upper portion 18b of the main solution tank 18 and the liquid disinfectant draining through the auger floor 74 into the lower portion 18a of the main solution tank.

Continuing with FIG. 7, a first nozzle 80a provides a flow of the liquid disinfectant into the lower portion 18a of the main solution tank to provide circulation of the liquid disinfectant, a second nozzle 80b provides a flow of the liquid disinfectant into the upper portion 18b of the auger end of the main solution tank 18, a third nozzle 80c provides a flow of the liquid disinfectant into the upper portion 18b of the main solution tank 18 near the auger end of the main solution tank 18 (i.e., where the auger 20 enters the main solution tank 18), and a fourth nozzle 80d is positioned opposite the auger end of the main solution tank 18 and provides a flow of the liquid disinfectant directed towards the auger screw 72.

A bubble tank assembly 128 is partially submerged in the disinfectant liquid below the static fluid level 78a and to preferably within approximately one half inch of the bottom of the main solution tank 18, and is further described in FIG. 8. A gas sample tube 129 resides in the main solution tank 18 and has a lower end above the static fluid level 78a, and is preferably between approximately six inches and approximately eight inches above the static fluid level 78a.

A second side view of the main solution tank 18 (an opposite side view from FIGS. 1 and 7) showing the continuous gas monitoring system 38, the pump 90, and liquid disinfectant generator elements are shown in FIG. 8. The pump 90 draws the liquid disinfectant from the lower portion 18a of the main solution tank 18 through the inlet line 92 and returns the liquid disinfectant to the nozzles 80a, 80b, 80c, and 80d (see FIG. 7) through nozzle lines 96a, 96b, 96c, and 96d respectively connected to the circulation pump 90 by the outlet line 94 through the manifold 104. The drain valve 98 is also connected to the outlet line 94, and a drain line 100 is connected to the drain valve 98 to allow convenient draining of the main solution tank 18. A neutralizer tank 130 is connected to a neutralizer nozzle 132 in, the drain line 100 by a neutralizer line 134. The neutralizer neutralizes the disinfectant liquid, and is preferably sodium sulfite.

The continuous gas monitoring system 38 measures the liquid disinfectant depth and concentration using the bubble tank assembly 128 and the gas sample tube 129 (also see FIG. 7). The continuous gas monitoring system 38 provides control signals over a control cable 122 to valves or pumps 116a, 116b, 116c, and 116d to control a flow of liquid disinfectant precursors from chemical tanks 114a, 114b, 114c, and 114d to a second manifold 112. The liquid disinfectant precursors preferably comprise an approximately 12 percent industrial clorox bleach (i.e., sodium hypochlorite), an approximately 12 percent to approximately 50 percent citric acid solution, an approximately 25 percent sodium chlorite solution as precursors for chlorine dioxide, and an anti-foam agent.

The continuous gas monitoring system 38 includes a continuous gas monitoring device which uses a diaphragm pump to provide the gas flow received through the gas sample tube 129 to a sensor. The sensor's electrical output is sent through a sensor circuit board to a digital panel meter which processes the sensor output and produces a digital readout in Parts Per Million (PPM) of the chemical levels in the liquid disinfectant. The continuous gas monitoring system 38 compares the measured gas level to the preset alarm levels and activates alarm indicators when gas levels exceed user set levels. If low gas levels are detected, a signal is sent to the liquid disinfectant generator to generate additional chlorine dioxide. If the liquid disinfectant is low, water is added to the systems. The continuous gas monitoring system 38 further includes data logging for recording data including chemical levels, fluid level, maintaining level, and kill ratio.

The static liquid level 78a (see FIG. 7) of the liquid disinfectant in the main solution tank 18 is measured using the bubble tank assembly 128 (see FIG. 7). The bubble tank assembly 128 comprises a six-inch cylinder sealed at the top with a one half inch tube protruding through the top of the seal and extending one half inch past the bottom of the cylinder. A second one half inch tube extends just through the seal into the top of the cylinder. The bubble tank assembly 128 is submerged in the liquid disinfectant in the main solution tank 18 to a depth wherein the longer tube is approximately one half-inch from the bottom of the main solution tank 18. Low-volume air is injected through the longer tube and the resulting pressure inside the cylinder is measured and converted to a measurement of depth of the liquid disinfectant in the main solution tank 18.

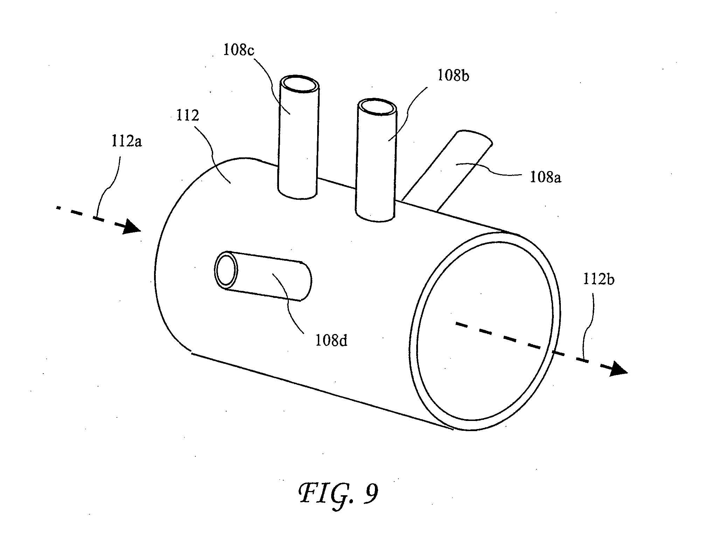

A detailed view of a preferred chemical manifold 112 is shown in FIG. 9. A first flow of liquid disinfectant 112a from the main solution tank 18 enters the manifold 112, and a second flow of liquid disinfectant 112b leaves the manifold 112 to enter the pump 90 (see FIG. 4). Chemical nozzles 108a, 108b, 108c, and 108d provide the liquid disinfectant precursors to the manifold 112. The liquid disinfectant precursors are thus introduced to a liquid disinfectant flow just prior to the flow entering the pump 90, where the liquid disinfectant precursors are mixed within the pump 90.

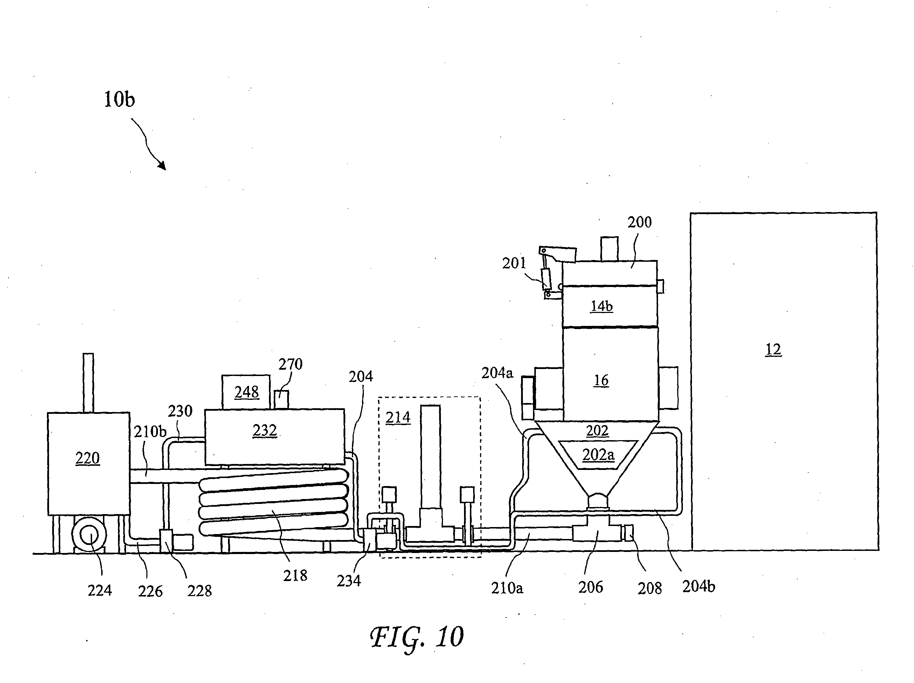

An overview of a second embodiment of a waste treatment system 10b according to the present invention is shown in FIG. 10. Several details of the waste treatment system 10b are not completely shown and are described in the following figures. The waste treatment system 10b includes a second feed hopper 14b equipped with a bridge breaking hopper ram 290 (see FIG. 11D) and shredder 16 of the first embodiment. The waste material is dumped into the feed hopper 14b, shredded by the shredder 16, and the shredded waste falls into a treatment hopper 202. An SS 316 L sub-plate 15 is preferably fitted between the feed hopper 14 and the shredder 16 with suitable gaskets residing on each side of the sub-plate. The feed hopper 14b preferably fits onto the sub-plate 15 and the sub-plate is fitted to the shredder 16.

The shredder 16 is described in FIGS. 3A, 3B, 3C, 4, and 5 above.

The treatment hopper 202 has downwardly inwardly sloped sides, an access port 202a, preferably an approximately 30 gallon capacity and is preferably at least approximately 24 inches high. The liquid disinfectant is carried to the treatment hopper 202 through disinfectant feed lines 204a and 204b and mixes with the shredded waste to produce a wetted slurry. The wetted slurry is fed from the treatment hopper 202 into a first "T" 206 and is advanced by a main pump 214 through a first wetted slurry line 210a into a dwell area 218. The dwell area 218 is thus in fluid cooperation with the wetting area 202 through the "T" 206, the line 210a, and the pump 214. The wetted slurry is advanced through the dwell area 218 at a rate resulting in a sufficient dwell period between wetting and de-wetting to disinfect the wetted slurry, which sufficient dwell time is preferably between approximately three minutes and approximately four minutes. The line 210a is preferably an approximately six inch diameter line, and is preferably made from stainless steel, and preferably 316L stainless steel. Following the dwell period in the dwell area 218, the wetted slurry advances through a second wetted slurry line 210b into a de-watering system 220, wherein the de-watering system 220 is in fluid communication with the dwell area 218 through the line 210b. The line 210b is preferably similar to the line 210a and is more preferably an approximately six inch diameter line, and is preferably made from stainless steel, and more preferably from 316L stainless steel. Once in the de-watering system 220, the wetted slurry is pressed to force substantially all of the liquid disinfectant out of the wetted slurry to produce disinfected dry waste. A waste conveyor system 224 carries the disinfected dry waste away from the de-watering system 220 for disposal. A preferred waste conveyer system 224 includes an auger for transporting the de-watered waste.

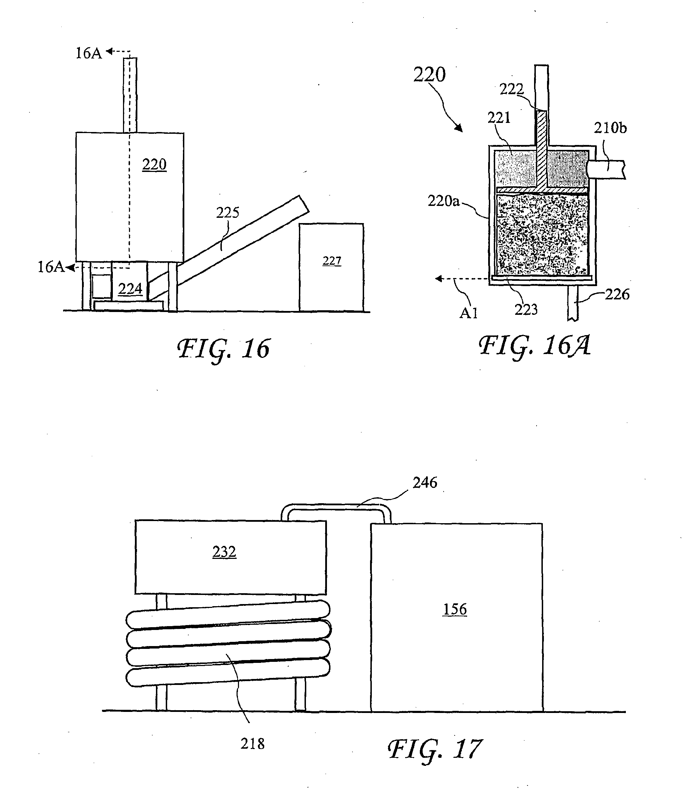

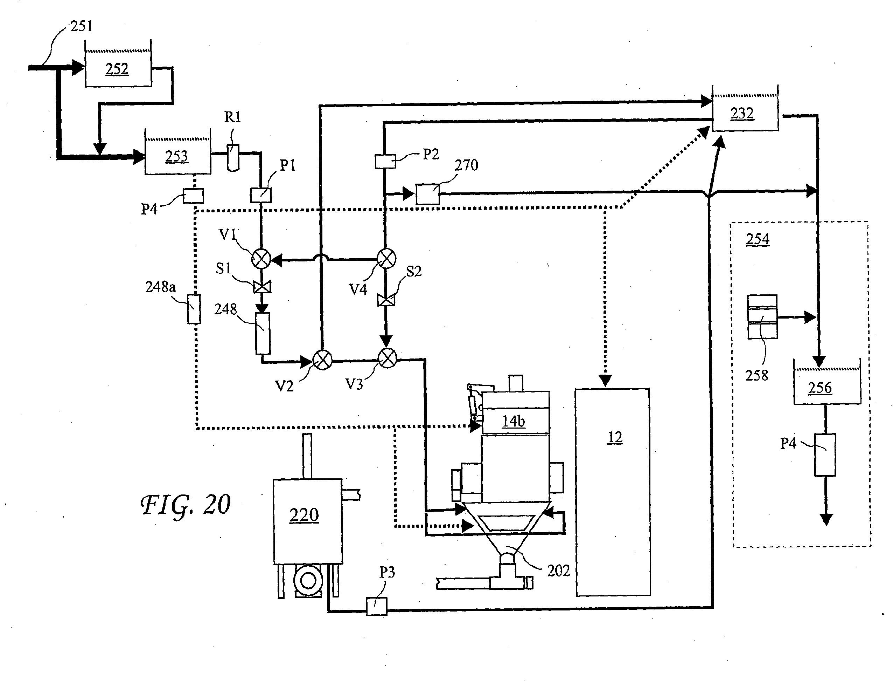

The main tank 232 stores newly generated liquid disinfectant for the liquid disinfectant generation system 248 and recycled liquid disinfectant from the de-watering system 220. The tank 232 preferably has a capacity to hold approximately 250 gallons, resides at high level wherein the top of the tank 232 is preferably at approximately 7.5 feet above the floor, and the tank 232 preferably includes at least one overflow connected to a waste water tank 256 (see FIG. 20). The main tank 232 is exhausted and exhaust plumbing is preferably sized for a velocity of up to approximately 700 feet per minute and the number of air changes is preferably six per hour. Air exhausted from main tank 232 is preferably exhausted to the feed hopper 14b. The liquid disinfectant level in main tank 232 is preferably monitored with an ultrasonic level sensor. A main tank 232 over flow is preferably positioned at a height to protect the ultrasonic sensor from being submerged in the liquid disinfectant. The ultrasonic sensor installation is preferably per manufacturers recommendations. Liquid disinfectant from the main tank 232 is pumped through the pump 234 (P2 in FIG. 17). The pump 234 preferably has a rating of approximately 30 gallons per minute at a head of approximately 12 Ft. Either side of the pump 234 may be fitted with valves in order to facilitate the pump's isolation and removal. The main tank 232 output flow is preferably pumped to both the liquid disinfectant generation system 248 and the treatment hopper 202 (see FIG. 20). The flow is preferably controlled by motorized valves and preferably set to flow concurrently to both the liquid disinfectant generation system 248 and to the treatment hopper 202, or all of the flow may be directed to the treatment hopper 202. Concurrent flow rates are preferably approximately eight gallons per minute through the liquid disinfectant generation system 248 and approximately 22 gallons per minute into the treatment hopper 202. When the entire flow is to the treatment hopper, preferably approximately 30 gallons per minute are fed from the main tank 232 into the treatment hopper 202.

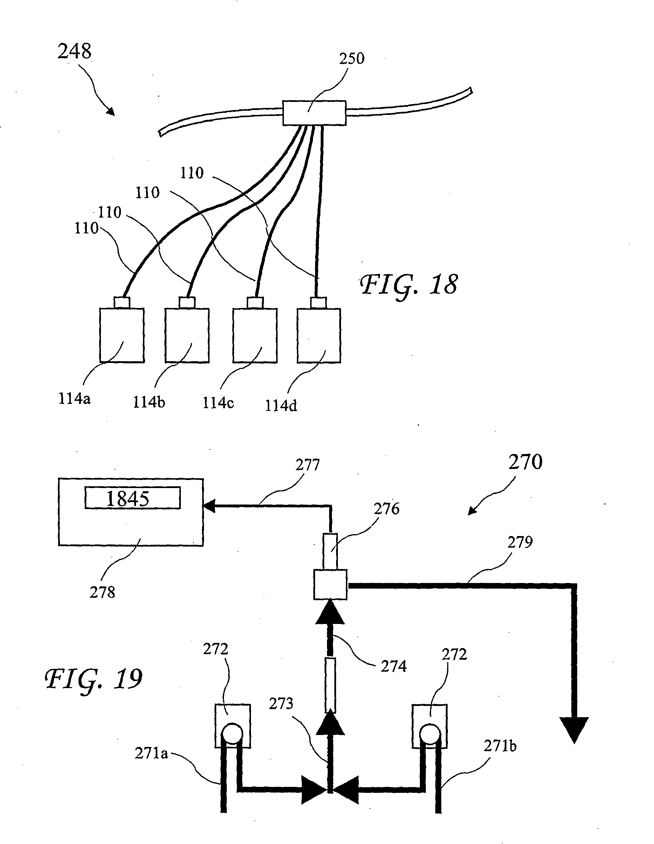

A temperature probe is preferably fitted to the main tank 232 at a level where the temperature probe is always submerged in liquid disinfectant. A flow from the down-stream side on the pump 234 (P2 in FIG. 20) is preferably supplied to the liquid disinfectant (preferably chlorine dioxide) monitoring system 270 (described in FIG. 19) and returned to the waste water tank 256. A refrigerant coil preferably resides in the main tank 232 and receives refrigerant from a refrigerant plant capable of reducing 250 gallons of water by 10 °F in 15 minutes. Materials in the cooling coil are preferably compatible with material selection as detailed below. An electrical heating element of at least approximately 15KW may be provided in the main tank 232 in order to maintain the liquid temperature within pre-set limits. System operation (for example, temperature) is preferably monitored and controlled by PLC to preset limits.

The dwell area 218 preferably has a capacity of at least approximately 14.5 Cubic Feet (approximately 110 Gallons). A preferred dwell area 218 is a coil comprising an approximately 6-inch diameter reinforced PVC hose, which is approximately 75 feet long. In general, the dwell area 218 is designed to ensure all floating waste particles are covered in liquid disinfectant (for example, remain in the slurry) for at least approximately three minutes to approximately four minutes, and is constructed of a material which is suitable for wetted parts. The length of the dwell area is thus a function of the volume pumping rate of the main pump 214, and is preferably at least the volume which the main pump 214 pumps in at least approximately three minutes to approximately four minutes. The coil configuration is preferred to reduce the floor space.

The liquid disinfectant extracted from the wetted slurry in the de-watering system 220 is filtered and passes through a de-watering system drain line 226, a de-watering system drain pump 228, and a main tank feed line 230 into the main tank 232. The liquid disinfectant may then pass through a disinfectant line 204 to a disinfectant feed pump 234 back through the disinfectant feed lines 204a and 204b and into the treatment hopper 202 to wet the shredded waste. A suitable pump 228 and/or 234 is an Iwaki Model MX-F401 supplied by Iwaki in Holliston, Massachusetts. The main feed tank 232 is preferably positioned above the dwell area 218 to conserve floor space and provide minimum separation of system elements.

A liquid disinfectant generation system 248 is provided for generating liquid disinfectant. The liquid disinfectant generation system 248 preferably resides on top of the main tank 232.

A wheeled bin 40 ready to lift is shown in FIG. 11A and the wheeled bin 40 with waste being poured into the second waste treatment system1Ob is shown in FIG. 11B. A lid 200 is provided to cover the feed hopper 14b. The lid 200 is generally closed but is opened by a cylinder 201 to allow waste material to be poured into the feed hopper 14b. When the lid 200 is closed, a lid latch 200a may be used to hold the lid firmly closed, which latch 200a is preferably controlled by the PLC. The lift system is otherwise as described in FIGS. 2A and 2B.

The capacity of the feed hopper 14b is preferably 25% bigger than a 95 US Gallon bin to allow for accepting the contents of the wheeled bin 40. A 95 Gallon wheeled bin 40 is nominally H 46.1 x W 27.7 x D 31.6 inches. During normal operation of the waste treatment system 10b, when a wheeled bin 40 is not being emptied into the feed hopper 14a, the lid 200 is preferably closed. When medical waste is being emptied into the feed hopper 14a, the lid 200 is opened as shown in FIG. 11B The lid 200 is preferably insulated with the equivalent of approximately 4 inches of Foam for noise abatement purposes. Both external and internal surfaces of the lid 200 are preferably made of a material which may be washed down with liquid containing bleach. The feed hopper 14b construction is preferably double walled and insulated with the equivalent of approximately 4 inches of foam. Both external and internal surfaces of the feed hopper 14b are preferably made of a material which can be washed down with liquid containing bleach. The lid 200 structure is preferably configured to prevent any splash back from the wheeled bin 40 finding its way to the outside of the feed hopper 14b.

The feed hopper 14b is preferably under a negative pressure and the exhaust is to be sized to achieve approximately 25 air changes per hour. The air intake to the feed hopper 14b is preferably filtered through a screen mesh filter and sized in order to keep air velocity below approximately 700 Ft. per minute. The air input is to be located on top of the lid 200 of the feed hopper 14b and protected from the hopper water / bleach wash down. The total volume between the closed lid 200 and an input hopper (i.e., the volume above the rotor and anvil) internal to the shredder 16 is preferably capable of holding the equivalent of two 95 US gallon wheeled bins, i.e. 24 cubic feet. A preferred Vecoplan Shredder has an input hopper capacity of 18 cubic feet which is part of the 24 cubic foot capacity. The hopper ram 290 (see FIG. 11D) is preferably provided for pushing the contents of the feed hopper 14b into the shredder 16.

The lid 200 of the feed hopper 14b may be opened for access to the shredder 16 for the purpose of clearing jams. Preferably, a pneumatically operated cylinder 201 is provided to open the lid 200, and mechanical locks are provided to hold the lid 200 open. When closed, the lid 200 is preferably held closed by the lid latch 200a. Electrical interlocks (or switches) may fitted to the lid 200 lift mechanism 201 so that when the lid 200 is open, the electrical interlocks will prevent operation of hopper ram 290 or the shredder 16.

An internal washer comprising an automatic bleach and water wash-down spray system may be fitted internally to the top of the lid 200. The spray is preferably a one to one mix of bleach and water. The internal washer includes a wash-down (or bleach) line 288 which connects a bleach tank 286 and a fresh water source to a spray nozzle in the feed hopper 14b. The spray nozzle preferably is positioned approximately six inches down from the top of the feed hopper 14b. The bleach tank 286 may be the same tank providing bleach for generating the liquid disinfectant (see FIG. 18). The internal washer preferably achieves complete coverage of all internal surfaces of the feed hopper 14b and may be adjustable to consume up to approximately six gallons per minute. The frequency and duration of this wash is user configurable in the PLC.

A more detailed side view of the lid 200 is shown in FIG. 11C, and a cross-sectional view of the lid 200 taken along line 11D-11D of FIG. 11C showing the hopper ram 290 is shown in FIG. 11D. The hopper ram 290 preferably comprises a hydraulically operated ram with a stroke to within approximately 2 inches of shredder rotor 50 of the shredder 16 (see FIGS. 3B and 4).

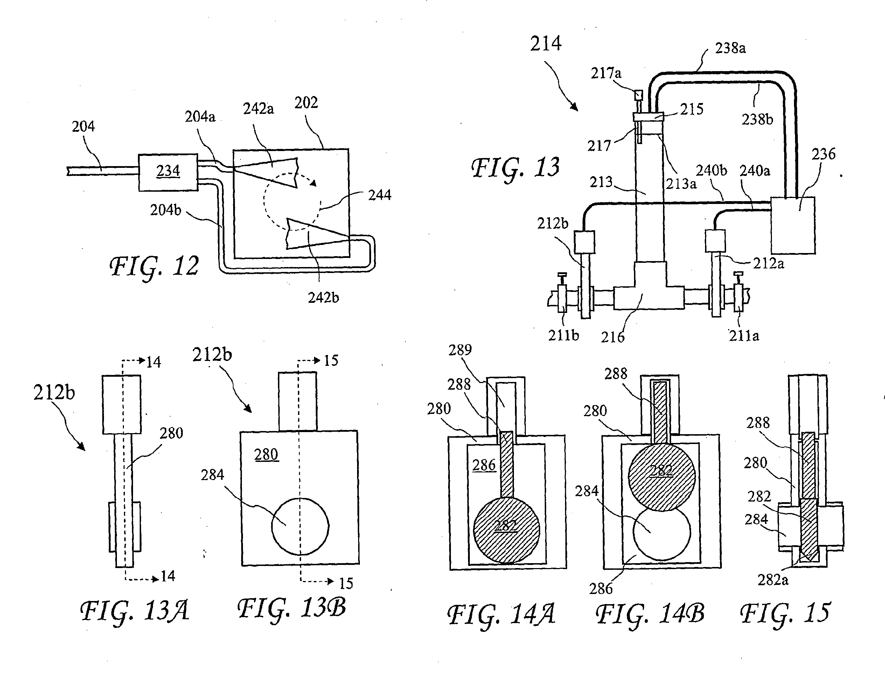

A top view of the treatment hopper 202 is shown in FIG. 12. The disinfectant feed lines 204a and 204b are laterally offset in opposite directions to provide sprays 242a and 242b respectively resulting in a swirl 244 inside the treatment hopper 202 to promote wetting of the shredded waste to generate a slurry. At all times, the total flow of liquid disinfectant into the treatment hopper 202 is preferably approximately 30 gallons per minute. The liquid disinfectant is preferably mixed with the shredded waste at a ratio of approximately one pound of shredded waste to approximately four pounds of liquid disinfectant. Such mixture results in a solution with a consistency of a slurry.

A common problem encountered in known hospital waste treatment systems is the difficulty in shredding all of the hospital waste components into small elements. Some hospital waste, such as titanium pins, may be bent and twisted, but are not cut or shredded into small pieces. These bent and twisted pins may foul or jam a pump used to advance the shredded and wetted slurry through the hospital waste treatment systems. A suitable pump should be capable of processing shards of metal up to 6 inches long without jamming and pump up to 5.5 cubic feet per minute to a head of 10 Ft.

A detailed view of a main pump 214 according to the present invention, used to advance the wetted slurry through the waste treatment system 10b is shown in FIG. 13. The pump 214 is preferably an air operated (i.e., pneumatic) pump comprising a displacement member 213 connected between two valves 212a and 212b. Vacuum and pressure lines 238a and 238b respectively connect between the displacement member 213 and a vacuum and/or pressure source 236. The vacuum and pressure lines 238a and 238b may be replaced by a single line providing both vacuum and pressure. The valves 212a and 212b are preferably gate valves and more preferably air operated (i.e., pneumatic) gate valves connected to the vacuum and/or pressure source 236 by lines 240a and 240b respectively. The pump 214 and the valves 212a, 212b are preferably connected by the vacuum/pressure lines 238a, 238b, 240a, and 240b to a computer controlled pneumatic controller. Additionally, manual valves 211a and 211b may be provided before (to the right of in FIG. 13) the valve 212a, and after (to the left of in FIG. 13) valve 212b, to close otherwise open ends of the line 210a if the pump 214 is removed from the waste treatment system 10b.

An example of a suitable pump is a Saniflow VC6 manufactured by Wilden Pump in Grand Terrace, California. The Saniflow VC6 pump includes the vertical column, valves, pressure and vacuum source, and controller.

The displacement member 213 may be empty or may include a piston to separate the vacuum/pressure from the wetted slurry. A preferred displacing member 213 is an empty vertical member, and more particularly an empty vertical cylinder. The preferred cylinder is no more than approximately six inches in diameter, and more preferably is approximately six inches in diameter. Such empty vertical member reduces the chances of fouling the pump with debris in the wetted slurry. A compressed air source of at least approximately eight bar and 15 cubic feet per minute and a vacuum source capable of displacing at least approximately five cubic feet per minute are preferably provided for the operation of the pump 214 and other requirements of the waste treatment system 10b.

A side view of the valve 212b is shown in FIG. 13A and a front view of the valve 212b is shown in FIG. 13B. The valves 212a and 212b include a valve body 280 and a passage 284. The valves 212a and 212b are preferably air operated gate valves. A cross-sectional view of the valve 212b taken along line 14-14 of FIG. 13A is shown with the valve 212b closed in FIG. 14A, and open in FIG. 14B. The valve 212b opens and closes by motion of a slide 282 actuated by a pneumatic piston 288. A side cross-sectional view of the valve 212b taken along line 15-15 of FIG. 13B is shown in FIG. 15 with the slide 282 in a closed position. The slide 282 has a somewhat pointed bottom 282a to assist in seating the slide 282 in the presence of solid material in the slurry. Such air operated gate valves provide reliable performance because jams are unlikely and any material which becomes lodged in the pump is likely to only reduce performance and do not prevent the valve from at least partially closing. Further, the valves are likely to clear without maintenance.

A preferred pump 214 is an air operated pump generally requiring vacuum and pressure, although the pump could include a piston biased by a spring, compressed air inside the pump, or the like, and only requires vacuum or pressure. Similarly, the pump 214 could be electrically operated using a solenoid to move a piston inside the pump 214. Vacuum, if required, is preferably generated from a vacuum pump of no more than 12-inch Hg with a swept volume of no less than 6.0 CFM. Pressure, if required, is preferably no more than 5 CFM of air at no more than 120 PSI. A safety valve may be fitted on a pressure output from the pump 214 and valve release pressure is preferably adjustable in a range approximately 20 pounds to approximately 60 pounds. A flow through the safety valve is preferably pumped into the treatment hopper 202.

A mesh 213a preferably resides proximal to the top of the displacement member 213 and extends below the surface of a liquid portion of the slurry by approximately six inches when the displacement member 213 is full, acting as a strainer to prevent solid material from passing. The mesh 213a is preferably made from SS 316 L and is preferably held in place by a clamp. A stainless steel tube 217 is preferably installed in the pump cap 215 and extends approximately six inches into the displacement member 213 and is terminated externally in an electrically operated ball valve 217a. The timing of the operation of the ball valve 217a may be synchronized with the operation of the pump 214. The synchronization is to enable a sample of the liquid disinfectant to be drawn off. The sample may be used for (after dilution) measurement of the concentration of C 102 in the liquid disinfectant (see FIG. 19).

A preferred pump 214 operates as follows. The wetted slurry is advanced into the pump 214 from the treatment hopper 202 by opening the inlet valve 212a, closing the outlet valve 212b, and applying vacuum to the displacement member 213, thereby sucking the wetted slurry from the treatment hopper 202 into the displacement member 213. The wetted slurry is then advanced toward the dwell area 218 by closing the inlet valve 212a, opening the outlet valve 212b, applying pressure to the displacement member 213.

A separate view of the de-watering apparatus 220 and waste conveyer system 224 is shown in FIG. 16. The waste conveyor system 224 carries the de-wetted waste away from the de-watering apparatus 220 and preferably facilitates dumping the de-wetted waste into standard waste compactor 227 or other waste container for disposal as regular garbage.

The de-watering system 220 is described as follows. The de-watering system is preferably capable of processing approximately 4 cubic feet per minute with an approximately 20 second cycle time to fill with slurry, de-water, and unload de-watered waste. The de-watering system 220 is preferably capable of de-watering to meet the "Paint Filter Liquid Test". The de-watering system 220 is preferably completely enclosed and any access points are preferably electrically interlocked to prevent system operation when an access point is open.

A cross-sectional view of the de-watering system 220 taken along line 16A-16A of FIG. 16 is shown in FIG. 16A. The de-watering system 220 receives wetted slurry from the dwell area 218 through the slurry line 210b and preferably uses a de-watering piston 222 pushed downward in a cylinder (or sieve) 221 to de-water the wetted slurry. The piston 222 is preferably pneumatically operated. The cylinder 221 is preferably approximately 18 inches in diameter and approximately 48 inches high. The walls of the cylinder 221 preferably have at least approximately 1500 approximately 1/16 inch diameter holes to allow the liquid disinfectant to escape as the piston 222 moves downward and to prevent waste particles from escaping the cylinder 221 (i.e., a filtering process) to preferably ensure that no solid particle greater than approximately 0.125 inches across can migrate from the de-watering system 220 through the pump 228 and into the main tank 232 (see FIG. 10). The holes are preferably beveled on the cylinder 221 exterior to reduce or prevent particles from becoming stuck in the holes. The liquid disinfectant flows down between the walls of the cylinder 221 and the outer housing 220a of the de-watering system 220 and is caught in the bottom of the de-watering system 220 and enters the line 226. A sliding plate 223 resides under the cylinder 221 and moves along arrow Al to close while the piston 222 moves downward, and to open after the piston 222 has reached its lowest point to release the de-watered waste into the waste conveyor system 224 to be removed.

The liquid disinfectant from the de-watering system is preferably pumped by pump 228 (see FIG. 10) to main tank 232. The pump 228 preferably has a capacity of approximately 30 gallons per minute to a head of approximately 10 ft. Valves are preferably fitted to both in-put and out-put from the pump 228 to facilitate removal and replacement of the pump.

Maintenance hatches are preferably provided around the de-watering system 220 to allow access for removal of the SS sieve filter, access to conveyor 225 at all locations, and any moving parts in the de-watering system. Locations covered with liquid disinfectant are preferably fabricated out of materials suitable for tanks. Where mechanical loads are experienced the materials are preferably metal. Wetted parts are preferably SS 316 L or an approved plastic.

The de-watering system 220 may be exhausted using an exhaust pipe preferably sized for a velocity of no more than approximately 700 Feet per minute and achieving approximately 25 air changes per hour.

De-watered waste from the de-watering system 220 is preferably fed to a covered conveyer (e.g., an auger) 225 (see FIG. 16) for transport to a waste compactor 227 or other waste storage or disposal means. The conveyer 225 is preferably a covered auger. The conveyer 225 is preferably located at one end of the waste treatment system 10b and hinged to allow de-watered waste to be directed either to the left or right of the waste treatment system 10b. The conveyer 225 is preferably approximately twelve feet long and the output end of the conveyer 225 is preferably adjustable in height to an overall height of approximately 9 ft and also capable of rotating horizontally through approximately 120° out the back of the de-watering system 220. The conveyer 225 is preferably constructed from SS 316 L. The output of the conveyor 225 is preferably fitted with an approximately 1.5 inch SS mesh which is manually adjustable into and out of the waste stream. The SS mesh facilitates the recovery of spore sample containers from the waste stream. The conveyer 225 is preferably capable of handling approximately four cubic feet of de-watered waste per minute and its operation is controlled by PLC, the parameters of which are software configurable by the user. A suitable conveyer 225 is a standard eight inch diameter auger.

A detailed view of the main tank 232 and a waste water tank 255 is shown in FIG. 17. The waste water tank 255 is connected to the main tank 232 by a waste water line 246. The waste water tank 255 catches an overflow from the main tank 232.

A detailed view of a preferred liquid disinfectant generation system 248 is shown in FIG. 18. The flow rate for fresh water into the liquid disinfectant generation system 248 is preferably controlled by a first flow regulator R1 (see FIG. 20). The range of adjustment of the flow regulator R1 is preferably between approximately six and approximately twelve gallons per minute. Re-circulated liquid disinfectant from the main tank 232 (see FIG. 20) will not have a flow regulator fitted but will be initially set, for example, by selecting an appropriate orifice when the system is first being commissioned. The water back pressure in the feed water supply to the liquid disinfectant generation system 248 is preferably monitored by the PLC against preset limits, which are software configurable by the user.

A preferred liquid disinfectant used in the water treatment system 10b is aqueous chlorine dioxide (CL02) and a preferred liquid disinfectant generation system 248 is a chlorine dioxide generation system. The aqueous chlorine dioxide may be generated by the following chemical reaction by means of a venturi:

NaOCl + 2 NaClO2 + 2 H3C6H507 -P 2 C102 + 3 NaCl + H20

Additionally, an anti-foaming agent is added diluted to a ratio of 1 part water to 1 part de-foaming agent. A suitable de-foaming agent is AF9010 made by GE Silicones in Wilton, Connecticut, or the like.

The preferred chlorine dioxide generation system is preferably at least approximately 65 % efficient and preferably capable of reliably producing a concentration of C102 from approximately 500 PPM to approximately 1,000 PPM, and more preferably capable of reliably producing a concentration of chlorine dioxide from approximately 500 PPM to approximately 700 PPM.

The preferred chlorine dioxide generation system comprises pre-cursor containers 114a, 114b, 114c, and 114d, pre-cursor lines 110, and an eductor 250 (or venturi) for combining the pre-cursors with either water or partially depleted aqueous chlorine dioxide. The pre-cursors are supplied to the eductor 250 by a siphoning action and no pumping is required. Line 110 lengths for each pre-cursor to the eductor 250 are preferably the same to an accuracy of +1-approximately three inches. A non-return valve with a filter is provided at the containers 114a, 114b, 114c and 114d end of each line 110. The containers 114a, 114b, and 114c, and 114d contain approximately 25 percent sodium chlorite solution, a 12 to 50 percent citric acid solution, and an approximately 12.5 percent industrial Clorox bleach (i.e. sodium hypochlorite) as pre-cursors for generating aqueous chlorine dioxide, and the de-foaming agent respectively. An example of a suitable eductor 250 is available from ChemCal in Grapevine, Texas. The back pressure in the water supply from the feed water tank 253 to the eductor 250 is preferably monitored by the PLC against preset limits, which limits are software configurable, by the user.

The chlorine dioxide generation is a mildly exothermic reaction and the overall process water temperature is to be monitored and controlled as follows. The feed water tank 253 may include a heater preferably comprising a thermostatically controlled heating element of at least 15KW fitted. The temperature of the feed water is automatically controllable to predetermined limits and this is configurable within the PLC. The main tank 232 preferably includes a heating element fitted to the base of the tank with a rating of at least 15KW. The temperature of the process water is to be automatically controllable to predetermined limits between 50 and 60 °F. A cooling element is preferably installed in the main tank 232 to be capable of cooling 250 gallons of water by 10 °F in a ten minute period. The operating temperature for the system is preferably less than 60 °F (15 °C).

A preferred liquid disinfectant monitoring system 270 comprising a chlorine dioxide monitoring system is described in FIG. 19. The preferred chlorine dioxide monitoring system comprises a 10:1 1 dilution system with two peristaltic pumps 272, a static mixer 274, special high range sensor and flowcell assembly 276, and a monitor with display 278. A sample flow of aqueous chlorine dioxide 271a has a sample flowrate of approximately three cubic centimeters per minute and a flow of dilution water 271b has a flowrate is approximately 30 cubic centimeters per minute. Each flow 271a and 271b is pumped by one of the peristaltic pumps 272 into the static mixer 274 and a mixed flow 273 flows from the static mixer to the sensor and flowcell assembly 276. A signal 277 from the sensor and flowcell assembly 276 is provided to the monitor with display 278. The monitor with display 278 preferably provides a read out in PPM over a 0-2000 range. An example of a suitable chlorine dioxide monitoring system 270 is a model Q45H/65-2-9 Chlorine Dioxide Monitor made by ATI, Inc. in Collegeville, Pennsylvania. The monitor with display 278 provides a control signal to the PLC, and the PLC controls the chlorine generation system 248.

A overview of liquid disinfectant flows in the waste treatment system 10b is described in FIG. 20. A feed water tank 253 stores water for use by the waste treatment system 10b. Water may be provided to the feed water tank 253 directly through a facility water supply line 251, or from a facility water tank 252 fed by the facility water supply line 251. The water supply to the feed water tank 253 preferably is capable of supplying approximately 300 gallons per hour and a peak flow of approximately 200 gallons in an approximately 15 minute period. Water from the feed water tank 253 is pumped through the flow regulator R1 by a first pump P1 through a first valve V1 to the liquid disinfectant generation system 248 where the water is combined with the pre-cursors to generate the liquid disinfectant used in the waste treatment system 10b. Additionally, depleted liquid disinfectant in the main tank 232 may be pumped by a second pump P2 through a fourth valve V4 and the valve V 1 through the liquid disinfectant generation system 248 to generate non-depleted liquid disinfectant A first shut-off valve Si resides between the valve V1 and the liquid disinfectant generation system 248 to allows the flow to the liquid disinfectant generation system 248 to be blocked.

The liquid disinfectant flow from the liquid disinfectant generation system 248 may flow through a second valve V2 and a third valve V3 to the treatment hopper 202 for wetting the shredded waste material, or the liquid disinfectant from the liquid disinfectant generation system 248 may flow through the second valve V2 and to the main tank 232. The liquid disinfectant may also be provided to the treatment hopper 202 from the main tank 232 through the pump P2, valve V4, and valve V3. A second shut-off valve 52 resided between the valves V3 and V4 to allow the flow from the main tank 232 to the treatment hopper 202 to be blocked. At all times, the preferred total flow of liquid disinfectant into the treatment hopper 202 is approximately 30 gallons per minute.

The flow through the liquid disinfectant generation system 248 is selectable from either fresh water from feed water tank 253 or partially depleted liquid disinfectant from the main tank 232, which already contains liquid disinfectant but is somewhat depleted. The depleted (or used) liquid disinfectant is preferably filtered by flowing through the holes in the cylinder 221 to prevent any particle greater than approximately 0.125 inches passing through liquid disinfectant generation system 248. The liquid disinfectant is provided to main tank 232 until a pre determined level is reached. The valve V1 controls the flow into the liquid disinfectant generation system 248 and allows selection of a depleted liquid disinfectant flow from the main tank 232 or fresh water from the feed water tank 253. The liquid disinfectant is provided to the treatment hopper 202 for 15 minutes after the last wheeled bin 40 has been loaded.

The feed water tank 253 is preferably made of plastic and is capable of storing approximately 50 gallons. The tank is supplied with water from a main water supply or from a separate facility header tank. The supply of water from the feed water tank 253 is preferably regulated by a flow regulator R1 in the flow from the feed water tank 253. The feed water tank 253 is preferably covered on top and totally insulated with a minimum of approximately four inches of rock wool or fiberglass. The feed water tank 253 will be fitted with an electrical heating element. The feed water tank 253 preferably includes an overflow which is approximately one inch in diameter and is fitted a minimum of approximately four inches above the nominal water level in the feed water tank 253. The feed water tank 253 overflow is pumped to the wastewater effluent treatment tank 256. The flow regulator R1 is preferably fitted to the feed water tank 253 approximately six inches above the nominal water level. An approximately ten gallon per minute resin bed water softener may be provided in some instances to soften the water entering the feed water tank 253.

The de-watering system 220 is connected to the main tank 232 through a third pump P3 (also shown as the de-watering system drain pump 228 in FIG. 10) to provide the liquid disinfectant reclaimed in the de-watering system 220 to the main tank 232 for reuse.

A waste water processing system 254 neutralizes and adjusts the PH of liquid disinfectant being released to a sanitary sewer. When the liquid disinfectant is aqueous chlorine dioxide, the neutralizer is preferably sodium thiosulfate (NaSO3), which neutralizes the aqueous chlorine dioxide producing a neutralized liquid. The chemical reaction exercised in the waste water processing system 254 when sodium thiosulfate is used as a neutralizer is:

5 NaS03 + 2 CL02 + H20 5 Na2SO4 + 2 HCL

The waste water tank 256 is connected to the main tank 232 by the waste water line 246 (see FIG. 17) to catch overflow from the main tank 232. A neutralizer from a neutralizer tank 258 is mixed with the flow into the waste water tank 256 and the mixture is held in the waste water tank 256 for a soak time to allow the neutralizer to neutralize the waste water. A preferred soak time of between approximately 30 minutes and approximately 45 minutes is used prior to pumping the neutralized liquid to a drain. The tank 256 is preferably fitted with both PH and temperature probes, and the PH may be adjusted to proper regulated levels using liquid caustic sodium. The tank 256 is preferably covered and an air outflow from the tank 256 may be sent to the treatment hopper 202. The dosage level of the various chemicals is preferably metered through peristaltic pumps. The resulting mixture is preferably mixed using compressed air agitation. The tank 256 preferably has capacities of approximately 400 gallons and is preferably covered and exhausted and ductwork is preferably sized at a speed of up to approximately 700 feet per minute with a preferred number of air changes per hour of 25.

A second water flow from the feed water tank 253 is pumped by a fourth pump P4 and through a second liquid disinfectant generation system 250a independently of the water supply to the liquid disinfectant generation system 248 to generate a second liquid disinfectant. Preferably, the second liquid disinfectant is a bleach and water mixture, and more preferably a 1:1 bleach and water mixture. The bleach may be the same bleach as used to generate the first liquid disinfectant, and the second liquid disinfectant generation system 250a may be a second eductor similar to the eductor 250, but only mixing bleach with a flow of water. A flow of the second liquid disinfectant may be provided to the waste handling section 12 to wash down waste containers, for example the wheeled bin 40, and such wash down may be automated. Additional flows of the second liquid disinfectant may be provided to the feed hopper 14b, the treatment hopper 202, and the main tank 232. The flows of the second liquid disinfectant are shown as dotted lines in FIG. 20.

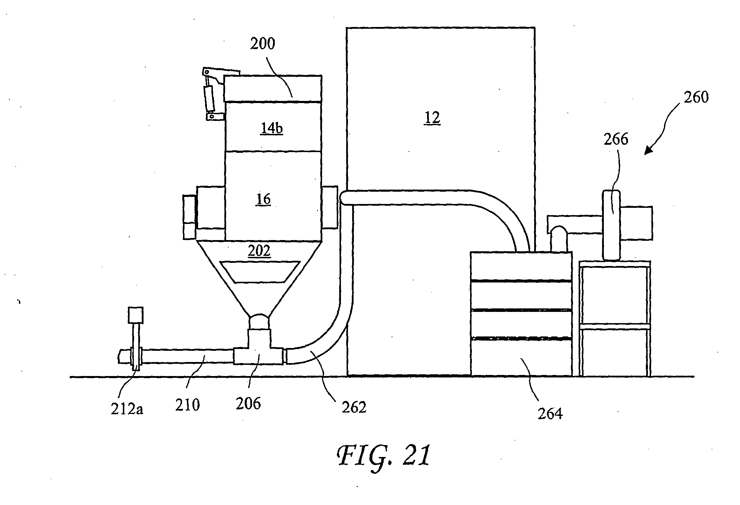

An add-on paper shredding system 260 is shown in FIG. 21. The paper shredding system 260 includes a vacuum hose 262, a barrel 264, and a vacuum unit 266. The cap 208 (see FIG. 10) is removed and the valve 212a is closed. The hose 262 is connected between the open end of the wetted slurry line 210 and the barrel 264. The vacuum unit 266 creates a vacuum in the barrel 264. When paper is dumped into the feed hopper 14b, the shredder 16 shreds the paper which falls through the treatment hopper 202 (now dry) and into the line 210. The shredded paper is then drawn through the hose 262 and into the barrel 264 and later disposed of. When the waste treatment system 10b is converted between treating general hospital waste and shredding paper, the interior of the feed hopper 14b, shredder 16, and the treatment hopper 202 are washed down with the liquid disinfectant, preferably aqueous chlorine dioxide, to prevent contamination of paper subsequently shredded. The feed hopper 14b and shredder 16 are preferably cleaned manually before switching to paper shredding. A dye may be sprayed into the shredded paper to provide additional security.

In an exemplar embodiment, the continuous liquid monitoring system 38 includes a special 10:1 1 dilution system with two peristaltic pumps, special high range sensor and flowcell assembly. The electrical control panel comprises a PLC control unit, variable frequency drives for the shreder and auger, motor starters for fan and pumps, 120VAC and 24VDC control voltage supplies. The pump control box comprises chemical concentration, receiving tank water level, main tank water level and air pressure controls, and a three light stack alarm enunciator. The operator console comprising a six inch touch screen Human Machine Interface (HMI) operator interface display, Start-Stop and Emergency Stop control operators, waste bin color detectors and weight scale. The hydraulic unit control box comprises hydraulic unit controls and position sensor junction terminal blocks.

All system functions are completely automatic and controlled by a Programmable Logic Controller (PLC) unit and the HMI display. All the operator needs to do is load the waste bin in the bin cage and press the start button on the operator console. The system will start functioning in a pre-programmed sequence. The complete process is monitored for time, water level, and chemical concentration by the PLC unit. Should any operating parameters deviate from normal, treatment is automatically halted and the control panel alerts an operator. The operation of the system can be monitored on the HMI display as explained below.

Before powering the system for the first time, the following checking steps are performed. Ensure all power cords are securely fastened (cord plugs preferably have a guide notch to prevent wrong connection of the plug to the receptacle, if a cord is unplugged, ensure that the system power is turned off, and re-plug). Check that the hydraulic unit oil level is normal. Ensure chemical containers are connected and are at least 30 percent full. Check for water leakages in the system. And lastly, ensure a water supply is present.

The waste treatment system 10 may be started by executing the following steps. Turn power on at the electrical panel (the 120VAC and 24VDC power indicator lights that are on the right side of the panel should light.) Make sure there is no alarm message on the HMI display and the 3-stack light is green. Check that the bin lift is at the extreme down position. If alarm messages are present, bring the lift down manually by using the touch buttons on the HMI display. Load the waste bin in the bin cage and make sure the cage door is firmly closed. Make sure that the E-Stop push button is released. If the green lamp on the operator console is not on, then press the Reset button. The green lamp should then turn on. And lastly, press the start button. The system will now start to run in the following sequence based on the color of the waste bin, Red = Medical waste, Grey = Cafeteria (non medical) waste. The HMI display will indicate the operating mode. If the mode must be set manually, the mode can be set on the HMI display Mode select page. The operation of the system may also be monitored on the HMI display as explained below.

An operating sequence for the waste treatment system 10 comprises the following steps. The lift will lift the waste bin and empty the contents into the hopper (there is a 5 sec delay at the top emptying position). After the bin lowers to the start position the shredder will start running. If in Medical mode, the waste particles will fall into the receiving tank located below the shredder, at the same time the receiving tank is filled with chlorine dioxide. The slurry of medical waste and chlorine dioxide is then pumped into the retention coil, where the waste will remain submerged for a minimum of 3 minutes. The waste is then pumped to the dewatering station where it is pressed and dewatered and extruded into an auger tank. The disinfected waste is then agued up and out to a compactor or waste bin. The circulation pumps will start running. The cycle time may be longer if there are hard substances in the waste. After the cycle time is over the system will stop.

The system will stop during the normal running cycle under the following conditions: water level is low or high; chemical level is low; air pressure is low; any one of the motors fault (overloading or other electrical problems); and E-Stop or Stop push button is pressed. The system will start running the cycle from the beginning when the alarm conditions are cleared and Start button is pressed.

Alarms are indicated by a bell flashing in the upper-right corner of the display when an alarm is activated. To go to the alarms screen, the alarm button on the lower right corner of all the display screens is touched. Alarms are presented in an alarm list with predefined alarm texts. The alarm list contains the latest alarms and is arranged in alarm group order according to definition, so that the latest alarms are shown at the top of the list. The number of times the alarm has been generated (if selected), the status of the alarm, the time it was activated, became inactive or was acknowledged, is shown for every alarm. Touching the acknowledge button accepts an alarm. If the alarm condition is already cleared, then the alarm message line will disappear after acknowledgment. If the alarm condition still exists, the message line will continue to display.

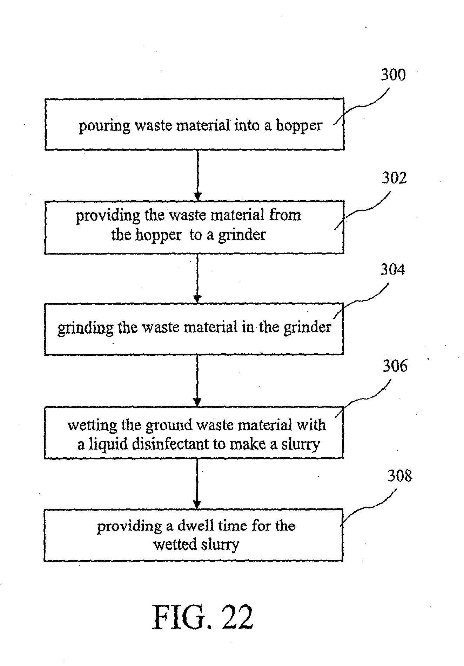

A method of waste treatment according to the present invention is described in FIG. 22. The method includes the steps of pouring waste material into a feed hopper at 300, providing the waste material from the feed hopper to a shredder at 302, shredding the waste material in the shredder at 304, wetting the shredded waste material with a liquid disinfectant to generate a wetted slurry at 306, and providing a dwell time for the wetted slurry of between two and three minutes at 308. In particular, providing a dwell time using the first waste treatment system 10a comprises carrying the wetted slurry on an auger, and providing a dwell time using the second waste treatment system 10b comprises advancing the wetted slurry through a dwell area.

More specifically, the method for waste treatment using the second waste treatment system 10b comprises an operator loading a wheeled bin 40 containing medical waste into the waste treatment system 10b, the operator pushing a start button, the wheeled bin 40 rising from a start position and emptying the medical waste into the feed hopper 14b, lowering the empty wheeled bin 40 to the start position, shredding the medical waste into confetti like particles in a shredder and allowing the shredded medical waste to fall into a wetting area under the shredder, adding aqueous chlorine dioxide to the wetting area and wetting the shredded waste with the aqueous chlorine dioxide in the wetting area to create a slurry comprising the shredded waste material and the aqueous chlorine dioxide, pumping the slurry through a dwell area to provide a dwell time wherein the wetted slurry remains immersed in the aqueous chlorine dioxide for at least approximately three minutes to disinfect the shredded waste, advancing the slurry into a de-watering apparatus, pressing the slurry to separate the aqueous chlorine dioxide from the disinfected waste, and moving the disinfected waste for compacting or into a waste bin.

Aqueous chlorine dioxide is preferably added to the shredded waste on the following basis. The PLC will monitor the weight of wheeled bins 40 entering into the feed hopper 14b within the previous approximately five minutes. The aqueous chlorine dioxide will be added to the solution at a rate of approximately 30 gallons per minute through a number of flow nozzles. The nozzles are recessed into the sidewalls of the treatment hopper 202. An ultrasonic level sensor monitors the level of liquid disinfectant in the treatment hopper 202 and controls the operation of the shredder 16. If the level of liquid disinfectant in the treatment hopper 202 drops below a threshold, the shredder 16 is stopped until the liquid disinfectant is replenished. The sensor trigger levels are adjustable via the system PLC. The sensor installation will be in accordance with manufacturer's recommendations. The waste treatment system 10b will continue to pump liquid disinfectant into the wetting hopper 202 for at least approximately 10 minutes after the last waste bin has been loaded to the waste treatment system 10b. This period of time is software configurable.

Where feasible, the concentration of chlorine dioxide in the liquid disinfectant will be measured in solution, or if measured in gaseous state, will be based on calibrated parameters. The measurement of the concentration of chlorine dioxide in solution is preferably achieved by accurately diluting a liquid disinfectant sample to a fixed predetermined concentration of approximately one part liquid disinfectant to ten parts of fresh water. Actual chlorine dioxide concentrations in undiluted liquid disinfectant are preferably in the range of approximately 200 to approximately 1,000 PPM and the subsequent dilution will result in a chlorine dioxide concentration somewhere between 0 and approximately 100 PPM. The chlorine dioxide measuring system comprises plumbing to provide a supply of fresh water from the facility water tank 252 to all chlorine dioxide sampling outlets, a filter using standard laboratory filter paper to provide a supply of liquid disinfectant solids removed, a peristaltic pump, which has an adjustable flow rate of approximately 0.06 to approximately 0.25 gallons per hour, for metering the filtered liquid disinfectant, an adjustable flow meter with a range of approximately 7 to approximately 15 gallons per hour for metering the flow of fresh water, a static mixer for mixing the fresh water with the liquid disinfectant (the static mixer having a Reynolds number between approximately 500 and approximately 1,000) to provide a mixture, a rotameter to maintain a constant flow rate of approximately 10 to approximately 20 gallons per hour of the mixture, a chlorine dioxide sensor with a measuring range of 0 to approximately 1000 PPM for measuring the concentration of chlorine dioxide in the mixture, and plumbing to carry the mixture to the water waste tank 255 for neutralizing and disposal. Samples of liquid disinfectant are preferably taken from the Weldon Pump and the main tank 232.

A PH meter may be provided to continuously monitor the liquid disinfectant at the waste water processing system 254. The specifications for the PH meter are monitoring range preferably from 0.00 to 14.00 PH between the temperatures 0 - 70°C with a resolution of 0.01 and an accuracy + 0.01 PH, calibration is to be 2 points, and the PH meter is to be suitable for interfacing with a data logger. The PH monitoring is preferably performed in the waste water processing system 254.

Temperatures within the waste treatment systems 10a and1Ob are preferably monitored with equipment capable of both °F or °C temperature readings, temperature range of -20°C to + 100°C, resolution of 0.1° to 1° to an accuracy of +0.75% + 1° C, and out-put suitable for data logging. The temperature monitoring is preferably performed in the main tank 232 and the waste water tank 256.

An exhaust system may be provided to extract air from around all liquid disinfectant surfaces. The exhaust system includes adjustable valves at all exhaust points to control flow. Exhaust speeds are preferably at least approximately 700 feet per minute and no more than approximately 1,000 feet per minute and the exhaust system preferably remains operational for approximately one hour after the waste treatment system has been switched off. Air is preferably extracted from the feed hopper 14b (preferably approximately 25 air changes per hour), the treatment hopper 202 (preferably approximately 6 air changes per hour), the de-watering system 220 (preferably approximately 25 air changes per hour), the main tank 232 (preferably approximately 6 air changes per hour), the waste water processing tank 255 (preferably approximately 25 air changes per hour), and all bulk chemical storage areas (preferably approximately at 6 changes per hour). Air from the exhaust system may be filtered through a wet Venturi scrubber. The air from the exhaust system is preferably released at high level to atmosphere, at least approximately 30 feet above ground level and 30 feet from the nearest building. Fan and exhaust system are preferably of plastic construction and suitable for exposure to C102 and rated for Zone 1 EExi EN50020 (Area of use European standard) and Class 1 Divisions 1 & 2 UL913 (Area of use US Standard). C102 concentration in exhaust gasses is preferably less than 0 1 PPM and is to be monitored by a suitable gas sensor. The C102 gas measuring system in the exhaust system is preferably capable of being calibrated and data logged.

The following general requirements apply to the waste treatment system 10b. Seals between surfaces are preferably fabricated out of FFKM (Perfluoroelastomer). These compounds contain fully fluorinated polymer chains and hence offer the excellent performance of elastomers when considering heat and chemical resistance and are for temperatures ranging from -15°C to +270° C. Piping and tubing used in the system construction are preferably selected from Chlorinated Poly (Vinyl Chloride) (CPVC), polyvinylidene fluoride (PVDF), and PTFE (Teflon). CPVC is a thermoplastic pipe and fitting material made with CPVC compounds which are commonly used for potable water distribution, corrosive fluid handling, and fire suppression systems. CPVC may be glued using PVC schedule 80 adhesive bonded with primer. PVDF possesses the characteristic stability of fluoropolymers when exposed to harsh thermal, chemical, and ultraviolet environments. PVDF is highly resistant to oxidizing agents and halogens and is almost completely resistant to aliphatic, aromatics, alcohols, acids, and chlorinated solvents. PTFE has low friction characteristics, excellent chemical resistance, is impervious to fungi or bacteria, has high temperature stability (260C), low temperature toughness (-160C), and good weathering resistance and electrical properties. Material used for tank fabrication is preferably 316 L SS with the welds cleaned and ground back on wetted surfaces, but may be other material preferably coated with one of CPVC, PVDF, and PTFE. In general, the parts exposed to aqueous chlorine dioxide are preferably made from PVDF, PTFE, or 316 L SS. The non-wetted parts may be made from any of CPVC,'PVDF, PTFE, or 316 L SS. The parts not exposed to aqueous chlorine dioxide may be made from material suitable for the material being stored.

### INDUSTRIAL APPLICABILITY

The present invention finds industrial applicability in the field of medical waste treatment.

## Claims

1. Apparatus for hospital waste treatment (10a, 10b), the apparatus comprising:
a feed hopper (14a, 14b) for receiving unprocessed waste material;
a shredder (16) residing below the feed hopper (14a, 14b) for receiving the unprocessed waste material from the feed hopper (14a, 14b) and shredding the unprocessed waste material;
a wetting area for receiving shredded material from the shredder (16);
liquid disinfectant in the wetting area (18, 202) to wet the shredded material to form a slurry;
a dwell area 21, 218) displaced from the wetting area and in fluid communication with the wetting area (18, 202) and providing a dwell time for the slurry advanced through the dwell area; and
a de-watering apparatus 26, 220) in fluid communication with the dwell area (21, 218) for receiving the slurry and for removing the liquid disinfectant from the slurry to generate de-watered waste,
wherein the dwell area comprises an auger (20) for carrying the slurry from the wetting area to the de-watering apparatus, **characterized in that**
a de-wetting area is fitted to the auger (20), wherein the liquid disinfectant used to wet the shredded waste is trapped and recirculated back into the main tank wherein the de-wetting area is in fluid cooperation with the auger (20) and comprises a rotatable section (26) of the auger housing (21) which may be rotationally positioned relative to an auger housing (21) at various rotations to adjust the position of a chute (24) and a fluid trap (28).

2. The apparatus of Claim 1, wherein the feed hopper (14a, 14b) includes a hopper ram (290) adapted to push the unprocessed waste material into the shredder (16).

3. The apparatus of Claim 2, wherein:
an opening lid (200) resides over the feed hopper (14a, 14b); and
the hopper ram is attached to the lid (200).

4. The apparatus of Claim 1, wherein the feed hopper (14a, 14b) includes a washdown nozzle (288) attached to a source of a bleach mixture (286), wherein the washdown nozzle (288) directs a spray of the bleach mixture into the interior of the feed hopper (14a, 14b).

5. The apparatus of Claim 1, wherein the shredder '16) includes:
an anvil (52),
a rotor (50) in cooperation with the anvil (52) to jointly shred the unprocessed waste material, the rotor (50) moveably mounted to allow the rotor (50) to move away from the anvil (52) if an object comes between the rotor (50) and anvil (52) thereby urging the rotor (50) away from the anvil (52);
a switch (88) which responsive to movement of the rotor (50), wherein the switch (88) removes power from the rotor (50) if the movement is sufficiently large; and
a shredder ram (66) adapted to push the unprocessed waste material towards the rotor (50), the ram automatically controlled to improve shredding efficiency.

6. The apparatus of Claim 1, wherein the dwell area (21, 218) comprises an auger (20) for carrying the slurry from the wetting area (18, 202) to the de-wattering apparatus (26, 226), wherein the auger (20) provides the dwell time based on the time a sample of the slurry spends in the auger (20).

7. The apparatus of Claim 1, wherein the dwell area (21, 218) comprises a coil and the dwell time is the time a sample of the slurry spends in the coil.

8. The apparatus of Claim 7, further including a main pump (214) for advancing the slurry from the wetting area (18, 202), through the coil, and into the de-wetting area.

9. The apparatus of Claim 8, wherein the pump (214) is an air operated pump.

10. The apparatus of Claim 1, wherein the de-wattering apparatus (26, 220) comprises:
a cylinder (221) in fluid cooperation with the dwell area (21, 218) and having a multiplicity of holes for allowing liquid disinfectant to escape the cylinder (221);
a de-wattering piston (222) positioned at the top of the cylinder (221) to advance downward through the cylinder (221) thereby compressing the slurry; and
a sliding plate (223) under the cylinder (221) having a closed position wherein the slurry is held to be de-watered by the de-watering piston (222) and an open position wherein the de-watered waste is released from the cylinder (221).

## Patentansprüche

1. Vorrichtung für die Behandlung von Krankenhausabfall (10a, 10b), wobei die Vorrichtung Folgendes umfasst:
einen Aufgabetrichter (14a, 14b) zum Aufnehmen von unverarbeitetem Abfallmaterial;
eine Zerkleinerungsvorrichtung (16), die sich unter dem Aufgabetrichter (14a, 14b) befindet, um das unverarbeitete Abfallmaterial von dem Aufgabetrichter (14a, 14b) aufzunehmen und das unverarbeitete Abfallmaterial zu zerkleinern;
einen Befeuchtungsbereich zum Aufnehmen von zerkleinertem Material von der Zerkleinerungsvorrichtung (16);
flüssiges Desinfektionsmittel in dem Befeuchtungsbereich (18, 202), um das zerkleinerte Material zu befeuchten, um einen Brei zu bilden;
einen Verweilbereich (21, 218), der von dem Befeuchtungsbereich verschoben ist und mit dem Befeuchtungsbereich (18, 202) in Fluidverbindung steht und
eine Verweilzeit für den durch den Verweilbereich voranbewegten Brei vorsieht; und
eine Entwässerungsvorrichtung (26, 220) in Fluidverbindung mit dem Verweilbereich (21, 218) zum Aufnehmen des Breis und zum Entfernen des flüssigen Desinfektionsmittels von dem Brei, um entwässerten Abfall zu erzeugen,
wobei der Verweilbereich eine Förderschnecke (20) zum Fördern des Breis von dem Befeuchtungsbereich zu der Entwässerungsvorrichtung umfasst, **dadurch gekennzeichnet, dass**
ein Entfeuchtungsbereich an der Förderschnecke (20) angebaut ist, wobei das zum Befeuchten des zerkleinerten Abfalls verwendete flüssige Desinfektionsmittel abgefangen und zu dem Hauptbehälter zurückgeführt wird, wobei der Entfeuchtungsbereich in Fluidzusammenwirkung mit der Förderschnecke (20) steht und einen drehbaren Abschnitt (26) des Förderschneckengehäuses (21) umfasst, der drehend an verschiedenen Drehungen relativ zu einem Förderschneckengehäuse (21) positioniert werden kann, um die Lage einer Rutsche (24) und eines Fluidabscheiders (28) zu verstellen.

2. Vorrichtung nach Anspruch 1, wobei der Aufgabetrichter (14a, 14b) einen Trichter-Druckstempel (290) umfasst, der dazu angepasst ist, das unverarbeitete Abfallmaterial in die Zerkleinerungsvorrichtung (16) zu drücken.

3. Vorrichtung nach Anspruch 2, wobei:
sich ein öffnender Deckel (200) über dem Aufgabetrichter (14a, 14b) befindet; und
der Trichter-Druckstempel an dem Deckel (200) angebracht ist.

4. Vorrichtung nach Anspruch 1, wobei der Aufgabetrichter (14a, 14b) eine Waschdüse (288) umfasst, die an einer Quelle eines Bleichegemischs (286) angebracht ist, wobei die Waschdüse (288) einen Sprühstrahl des Bleichegemischs in das Innere des Aufgabetrichters (14a, 14b) richtet.

5. Vorrichtung nach Anspruch 1, wobei die Zerkleinerungsvorrichtung (16) Folgendes umfasst:
einen Amboss (52),
einen Rotor (50), der mit dem Amboss (52) zusammenwirkt, um gemeinsam das unverarbeitete Abfallmaterial zu zerkleinern, wobei der Rotor (50) beweglich montiert ist, um zuzulassen, dass sich der Rotor (50) von dem Amboss (52) weg bewegt, wenn ein Gegenstand zwischen den Rotor (50) und den Amboss (52) gelangt und dadurch den Rotor (50) von dem Amboss (52) weg drängt;
einen Schalter (88), der auf Bewegung des Rotors (50) anspricht, wobei der Schalter (88) Leistung von dem Rotor (50) entfernt, wenn die Bewegung ausreichend groß ist; und
einen Zerkleinerungsvorrichtungs-Druckstempel (66), der dazu angepasst ist, das unverarbeitete Abfallmaterial zum Rotor (50) zu drücken, wobei der Druckstempel automatisch gesteuert wird, um den Zerkleinerungswirkungsgrad zu verbessern.

6. Vorrichtung nach Anspruch 1, wobei der Verweilbereich (21, 218) eine Förderschnecke (20) umfasst, um den Brei von dem Befeuchtungsbereich (18, 202) zu der Entwässerungsvorrichtung (26, 220) zu fördern, wobei die Förderschnecke (20) die Verweilzeit basierend auf der Zeit vorsieht, die eine Probe des Breis in der Förderschnecke (20) verbringt.

7. Vorrichtung nach Anspruch 1, wobei der Verweilbereich (21, 218) eine Spirale umfasst und die Verweilzeit die Zeit ist, die eine Probe des Breis in der Spirale verbringt.

8. Vorrichtung nach Anspruch 7, weiter umfassend eine Hauptpumpe (214) zum Voranbewegen des Breis von dem Befeuchtungsbereich (18, 202), durch die Spirale und in den Entfeuchtungsbereich.

9. Vorrichtung nach Anspruch 8, wobei es sich bei der Pumpe (214) um eine luftbetriebene Pumpe handelt.

10. Vorrichtung nach Anspruch 1, wobei die Entwässerungsvorrichtung (26, 220) Folgendes umfasst:
einen Zylinder (221) in Fluidzusammenwirkung mit dem Verweilbereich (21, 218) und mit einer Vielzahl von Löchern zum Zulassen, dass flüssiges Desinfektionsmittel aus dem Zylinder (221) austritt;
einen Entwässerungskolben (222), der oben an dem Zylinder (221) positioniert ist, um sich nach unten durch den Zylinder (221) voranzubewegen und dadurch den Brei zu verdichten; und
eine Gleitplatte (223) unter dem Zylinder (221) mit einer geschlossenen Stellung, in der der Brei gehalten wird, um von dem Entwässerungskolben (222) entwässert zu werden und einer offenen Stellung, in der der entwässerte Abfall aus dem Zylinder (221) freigesetzt wird.

## Revendications

1. Appareil de traitement de déchets hospitaliers (10a, 10b), l'appareil comportant :
une trémie d'alimentation (14a, 14b) destinée à recevoir des déchets non traités ;
un broyeur (16) se trouvant sous la trémie d'alimentation (14a, 14b) destiné à recevoir les déchets non traités en provenance de la trémie d'alimentation (14a, 14b) et destiné à broyer les déchets non traités ;
une zone de mouillage destinée à recevoir des déchets broyés en provenance du broyeur (16) ;
du désinfectant liquide dans la zone de mouillage (18, 202) destiné à mouiller les déchets broyés pour former des boues ;
une zone d'attente (21, 218) déplacée par rapport à la zone de mouillage et en communication fluidique avec la zone de mouillage (18, 202) et procurant un temps d'attente pour les boues avancées au travers de la zone d' attente ; et
un appareil de déshydratation (26, 220) en communication fluidique avec la zone d'attente (21, 218) destiné à recevoir les boues et destiné à retirer le désinfectant liquide des boues pour produire des déchets déshydratés,
dans lequel la zone d'attente comporte une vis sans fin (20) destinée à transporter les boues depuis la zone de mouillage jusqu'à l'appareil de déshydratation, **caractérisé en ce que**
une zone de démouillage est installée sur la vis sans fin (20), dans lequel le désinfectant liquide utilisé pour mouiller les déchets broyés est pris au piège et envoyé de retour dans le réservoir principal, dans lequel la zone de démouillage est en coopération fluidique avec la vis sans fin (20) et comporte une section rotative (26) du logement (21) de la vis sans fin qui peut être positionnée de manière rotative par rapport à un logement (21) de la vis sans fin à différentes rotations afin de régler la position d'une goulotte (24) et d'un piège à fluide (28).

2. Appareil selon la revendication 1, dans lequel la trémie d'alimentation (14a, 14b) comprend un piston (290) de trémie adapté pour pousser les déchets non traités dans le broyeur (16).

3. Appareil selon la revendication 2, dans lequel :
un couvercle ouvrant (200) se trouve au-dessus de la trémie d'alimentation (14a, 14b) ; et
le piston de trémie est attaché au couvercle (200).

4. Appareil selon la revendication 1, dans lequel la trémie d'alimentation (14a, 14b) comprend une buse de lavage à grande eau (288) attachée à une source d'un mélange de blanchiment (286), dans lequel la buse de lavage à grande eau (288) oriente une pulvérisation du mélange de blanchiment dans la partie intérieure de la trémie d'alimentation (14a, 14b).

5. Appareil selon la revendication 1, dans lequel le broyeur (16) comprend :
une enclume (52),
un rotor (50) en coopération avec l'enclume (52) pour broyer ensemble les déchets non traités, le rotor (50) étant monté de manière mobile pour permettre au rotor (50) de s'éloigner de l'enclume (52) si un objet vient se placer entre le rotor (50) et l'enclume (52) pour de ce fait solliciter le rotor (50) à s'éloigner de l'enclume (52) ;
un commutateur (88) qui réagit au mouvement du rotor (50), dans lequel le commutateur (88) coupe toute alimentation électrique du rotor (50) si le mouvement est suffisamment important ; et
un piston (66) de broyeur adapté pour pousser les déchets non traités vers le rotor (50), le piston étant automatiquement commandé pour améliorer l'efficacité du broyage.

6. Appareil selon la revendication 1, dans lequel la zone d'attente (21, 218) comporte une vis sans fin (20) à des fins de transport des boues depuis la zone de mouillage (18, 202) jusqu'à l'appareil de déshydratation (26, 220), dans lequel la vis sans fin (20) procure le temps d'attente en fonction du temps qu'un échantillon de boues passe dans la vis sans fin (20).

7. Appareil selon la revendication 1, dans lequel la zone d'attente (21, 218) comporte une bobine et le temps d'attente est le temps qu'un échantillon des boues passe dans la bobine.

8. Appareil selon la revendication 7, comprenant par ailleurs une pompe principale (214) destinée à faire avancer les boues depuis la zone de mouillage (18, 202), au travers de la bobine, et jusque dans la zone de démouillage.

9. Appareil selon la revendication 8, dans lequel la pompe (214) est une pompe à air comprimé.

10. Appareil selon la revendication 1, dans lequel l'appareil de déshydratation (26, 220) comporte :
un cylindre (221) en coopération fluidique avec la zone d'attente (21, 218) et ayant une multiplicité de trous permettant au désinfectant liquide de s'échapper du cylindre (221) ;
un piston de déshydratation (222) positionné sur la partie supérieure du cylindre (221) à des fins d'avance vers le bas au travers du cylindre (221) pour de ce fait comprimer les boues ; et
un plateau coulissant (223) sous le cylindre (221) ayant une position fermée dans laquelle les boues sont retenues à des fins de déshydratation par le piston de déshydratation (222) et une position ouverte dans laquelle les déchets déshydratés sont libérés du cylindre (221).
